# EUROPÄISCHE PATENTSCHRIFT

(11) **EP 1 753 729 B1**
(45) Veröffentlichungstag und Bekanntmachung des Hinweises auf die Patenterteilung: **11.04.2012**
(21) Anmeldenummer: 05736446.5
(22) Anmeldetag: 06.05.2005
(51) Int. Cl.: C07D 233/90, C07D 487/04

(54) **NEUE IMIDAZOLDERIVATE, DEREN HERSTELLUNG UND DEREN VERWENDUNG ALS INTERMEDIATE ZUR HERSTELLUNG VON ARZNEIMITTELN UND PESTIZIDEN**
NOVEL IMIDAZOLE DERIVATIVES, THE PRODUCTION THEREOF, AND THE USE OF THE SAME AS INTERMEDIATE PRODUCTS FOR PRODUCING MEDICAMENTS AND PESTICIDES
NOUVEAUX DERIVES D'IMIDAZOLE, LEUR PRODUCTION ET LEUR UTILISATION EN TANT QUE PRODUITS INTERMEDIAIRES DANS LA PRODUCTION DE MEDICAMENTS ET DE PESTICIDES

(30) Priorität: 10.05.2004 DE 102004022970
(43) Veröffentlichungstag der Anmeldung: 21.02.2007
(73) Patentinhaber: Boehringer Ingelheim International GmbH, 55216 Ingelheim (DE); Boehringer Ingelheim Pharma GmbH & Co. KG, 55216 Ingelheim (DE)
(72) Erfinder: ECKHARDT, Matthias, 88400 Biberach (DE)
(74) Vertreter: Hammann, Heinz
(86) Internationale Anmeldenummer: PCT/EP2005/004942
(87) Internationale Veröffentlichungsnummer: WO 2005/110999

(56) Entgegenhaltungen:
- WO-A-03/104229
- WO-A-2004/050658
- US-A1- 2005 026 921

## Beschreibung

Gegenstand der vorliegenden Erfindung sind neue substituierte imidazole der allgemeinen Formel 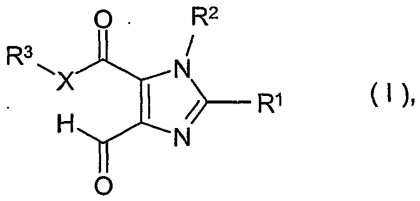 deren Tautomere, deren Enantiomere, deren Diastereomere, deren Gemische und deren Salze sowie Verfahren zu deren Herstellung. Die Verbindungen können als Intermediate zur Darstellung von Arzneimitteln oder Pestiziden Verwendung finden. Ausgehend von ihnen lassen sich weitere heterocyclische Ringe an das Imidazol zum Aufbau von wichtigen polycyclischen Grundstrukturen anknüpfen. Sie eignen sich im besonderen zur Darstellung von Imidazo[4,5-d]pyridazin-4-onen und lmidazo[4,5-c]pyridin-4-onen, die z.B. als DPP IV-Inhibitoren in der Behandlung von Diabetes eingesetzt werden können (s. z.B. WO 03/104229). Des Weiteren können breite Substituentenvariationen an drei Positionen des Imidazols unter unterschiedlichen Reaktionsbedingungen vorgenommen werden, welches diese Verbindungen besonders attraktiv als Grundbaustein (Scaffold) in der Synthese von kombinatorischen Bibliotheken erscheinen lässt.

In der obigen Formel I bedeuten
R¹ ein Fluor-, Chlor-, Brom- oder lodatom,
R² eine C₃₋₈-Alkylgruppe,
eine durch eine Gruppe Rₐ substituierte C₁₋₃-Alkylgruppe, wobei
Rₐ eine gegebenenfalls durch eine oder zwei C₁₋₃-Alkylgruppen substituierte C₃₋₇-Cycloalkylgruppe,
eine gegebenenfalls durch eine oder zwei C₁₋₃-Alkylgruppen substituierte C₃₋₈-Cycloalkenylgruppe,
eine Arylgruppe oder
eine Heteroarylgruppe,
eine C₃₋₈-Alkenylgruppe,
eine durch ein Fluor-, Chlor- oder Bromatom oder durch eine Trifluormethylgruppe substituierte C₃₋₆-Alkenylgruppe,
eine C₃₋₈-Alkinylgruppe,
eine Arylgruppe oder
eine Aryl-C₂₋₄-alkenylgruppe,
X ein Sauerstoff- oder Schwefelatom oder
ein Stickstoffatom, das durch R_{b} substituiert ist, wobei
R_{b} ein Wasserstoffatom,
eine Hydroxy-, Aryloxy-, Arylmethyloxy-, Heteroaryloxy-, Heteroarylmethyloxy-oder C₁₋₁₀-Alkyloxygruppe, wobei die Wasserstoffatome der Alkyloxygruppe ganz oder teilweise durch Fluoratome ersetzt sein können,
eine C₁₋₁₀-Alkylcarbonyl-, Arylcarbonyl-, Heteroarylcarbonyl-, C₁₋₁₀-Alkyloxycarbonyl-, C₁₋₁₀-Alkylaminocarbonyl-, Di-(C₁₋₁₀-alkyl)-aminocarbonyl-, C₁₋₁₀-Alkylsulfonyl-, Arylsulfonyl-, Heteroarylsulfonyl-, C₁₋₁₀-Alkylsulfinyl-, Arylsulfinyl-oder Heteroarylsulfinylgruppe, wobei die Wasserstoffatome der vorstehend genannten C₁₋₁₀-Alkylreste ganz oder teilweise durch Fluoratome ersetzt sein können,
eine C₁₋₁₀-Alkyl-, C₂₋₁₀-Alkenyl-, C₂₋₁₀-Alkinyl-, C₃₋₇-Cycloalkyl- oder C₅₋₈-Cyclo-alkenylruppe, wobei die Wasserstoffatome in den vorstehend genannten Gruppen jeweils ganz oder teilweise durch Fluoratome ersetzt sein können und wobei in den vorstehend genannten Gruppen jeweils 1 bis 4 Methylengruppen durch ein Sauerstoff- oder Schwefelatom, durch eine -NH- oder -N(C₁₋₃-Alkyl)- Gruppe oder durch eine Carbonyl-, Sulfinyl- oder Sulfonylgruppe ersetzt sein können,
eine Arylgruppe oder
eine Heteroarylgruppe bedeutet,
oder
R_{b} und R³ miteinander verknüpft und am Stickstoffatom zu einem Ring geschlossen sind, wobei R_{b} und R³ zusammen
eine C₂₋₇-Alkylengruppe, wobei eine oder zwei Methylengruppen jeweils durch ein oder zwei Fluoratome substituiert oder durch ein Sauerstoff-oder Schwefelatom, durch eine -NH- oder -N(C₁₋₃-Alkyl)-Gruppe oder durch eine Carbonyl-, Sulfinyl- oder Sulfonylgruppe ersetzt sein können,
oder eine C₄₋₇-Alkenylengruppe, wobei eine oder zwei Methylengruppen jeweils durch ein oder zwei Fluoratome substituiert oder durch ein Sauerstoff oder Schwefelatom, durch eine -NH- oder -N(C₁₋₃-Alkyl)-Gruppe oder durch eine Carbonyl-, Sulfinyl- oder Sulfonylgruppe ersetzt sein können und wobei die Doppelbindung Teil einer an den Ring anellierten Aryl- oder Heteroarylgruppe sein kann, bedeuten,
und
R³ ein Wasserstoffatom,
eine C₁-₂₀-Alkylgruppe, in der die Wasserstoffatome ganz oder teilweise durch Fluoratome ersetzt sein können und in der 1 bis 6 Methylengruppen jeweils durch ein Sauerstoff- oder Schwefelatom, durch eine -NH- oder -N(C₁₋₃-Alkyl)- Gruppe oder durch eine Carbonyl-, Sulfinyl- oder Sulfonylgruppe ersetzt sein können,
eine durch eine Gruppe R_{c} substituierte C₁₋₁₂-Alkylgruppe, in der die Wasserstoffatome ganz oder teilweise durch Fluoratome ersetzt sein können und in der 1 bis 4 Methylengruppen jeweils durch ein Sauerstoff- oder Schwefelatom, durch eine -NH-oder -N(C₁₋₃-Alkyl)-Gruppe oder durch eine Carbonyl-, Sulfinyl- oder Sulfonylgruppe ersetzt sein können, wobei
R_{c} eine gegebenenfalls durch eine oder zwei C₁₋₃-Alkylgruppen substituierte C₃₋₁₈-Cycloalkylgruppe, in der die Wasserstoffatome ganz oder teilweise durch Fluoratome ersetzt sein können und in der ein bis zwei Methylengruppen jeweils durch ein Sauerstoff- oder Schwefelatom, durch eine -NH- oder-N(C₁-₃-Alkyl)- Gruppe oder durch eine Carbonyl-, Sulfinyl- oder Sulfonylgruppe ersetzt sein können,
eine gegebenenfalls durch eine oder zwei C₁₋₃-Alkylgruppen substituierte C₅₋₁₈-Cycloalkenylgruppe, in der
die Wasserstoffatome ganz oder teilweise durch Fluoratome ersetzt sein können,
ein bis zwei Methylengruppen jeweils durch ein Sauerstoff- oder Schwefelatom, durch eine -NH- oder -N(C₁₋₃-Alkyl)- Gruppe oder durch eine Carbonyl-, Sulfinyl- oder Sulfonylgruppe ersetzt sein können und
die Doppelbindung Bestandteil einer an den Ring anellierten Aryl- oder Heteroarylgruppe sein kann,
eine Arylgruppe oder
eine Heteroarylgruppe bedeutet,
eine C₃₋₁₈-Cycloalkylgruppe, in der die Wasserstoffatome ganz oder teilweise durch Fluoratome ersetzt sein können und in der 1 bis 6 Methylengruppen jeweils durch ein Sauerstoff- oder Schwefelatom, durch eine -NH- oder -N(C₁₋₃-Alkyl)- Gruppe oder durch eine Carbonyl-, Sulfinyl- oder Sulfonylgruppe ersetzt sein können,
eine C₃₋₂₀-Alkenylgruppe, in der die Wasserstoffatome ganz oder teilweise durch Fluoratome ersetzt sein können und in der 1 bis 6 Methylengruppen jeweils durch ein Sauerstoff- oder Schwefelatom, durch eine -NH- oder -N(C₁₋₃-Alkyl)- Gruppe oder durch eine Carbonyl-, Sulfinyl- oder Sulfonylgruppe ersetzt sein können,
eine C₅₋₂₀-Cycloalkenylgruppe, in der die Wasserstoffatome ganz oder teilweise durch Fluoratome ersetzt sein können und in der 1 bis 4 Methylengruppen jeweils durch ein Sauerstoff- oder Schwefelatom, durch eine -NH- oder -N(C₁₋₃-Alkyl)-Gruppe oder durch eine Carbonyl-, Sulfinyl- oder Sulfonylgruppe ersetzt sein können, und in der die Doppelbindung Bestandteil einer an den Ring anellierten Aryl- oder Heteroarylgruppe sein kann,
eine C₃₋₂₀-Alkinylgruppe, in der die Wasserstoffatome ganz oder teilweise durch Fluoratome ersetzt sein können und in der 1 bis 6 Methylengruppen jeweils durch ein Sauerstoff- oder Schwefelatom, durch eine -NH- oder -N(C₁₋₃-Alkyl)- Gruppe oder durch eine Carbonyl-, Sulfinyl- oder Sulfonylgruppe ersetzt sein können,
eine Arylgruppe,
eine Heteroarylgruppe,
eine Aryl-C₂₋₆-alkenylgruppe
oder, sofern X kein Stickstoffatom, das durch eine Hydroxy-, Aryloxy-, Arylmethyloxy-, Heteroarytoxy-, Heteroarylmethyloxy- oder C₁₋₁₀-Alkyloxygruppe substituiert ist, darstellt, auch eine Aminogruppe, die durch eine oder zwei C₁₋₃-Alkylgruppen substituiert sein kann,
oder, ebenfalls sofern X kein Stickstoffatom, das durch eine Hydroxy-, Aryloxy-, Arylmethyloxy-, Heteroaryloxy-, Heteroarylmethyloxy- oder C₁₋₁₀-Alkyloxygruppe substituiert ist, darstellt, auch eine 3- bis 7-gliedrigen Cycloalkyleniminogruppe, wobei ein bis zwei Methylengruppen der Cycloalkyleniminogruppe jeweils durch ein Sauerstoffatom oder eine Carbonyl-, Sulfinyl- oder Sulfonylgruppe ersetzt sein können, oder
R³ und X zusammen bedeuten ein Fluor- oder Chloratom,
wobei unter den bei der Definition der vorstehend genannten Reste erwähnten Arylgruppen Phenyl- oder Naphthylgruppen zu verstehen sind, welche unabhängig voneinander durch Fluor- und Chloratome ein- bis fünffach substituiert und durch R_{d} mono-, di- oder trisubstituiert sein können, wobei die Substituenten gleich oder verschieden sein können und R_{d} ein Brom- oder lodatom, eine Trifluormethyl-, Cyan-, Nitro-, Amino-, Aminocarbonyl-, Aminosulfonyl-, Methylsulfonyl-, Acetylamino-, Methylsulfonylamino-, C₁₋₄-Alkyl-, C₁₋₃-Alkyl-carbonyl-, Cyclopropyl-, Ethenyl-, Ethinyl-, Hydroxy-, C₁₋₄-Alkyloxy-, C₁₋₄-Alkoxy-carbonyl-, Methylsulfinyl-, Phenylsulfinyl-, Methylsulfonyl-, Phenylsulfonyl-, Difluormethoxy- oder Trifluormethoxygruppe darstellt,
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Chinolinyl- oder Isochinolinylgruppe
oder eine Pyrrolyl-, Furanyl-, Thienyl- oder Pyridylgruppe in der eine oder zwei Methingruppen durch Stickstoffatome ersetzt sind,
oder eine Indolyl-, Benzofuranyl-, Benzothiophenyl-, Chinolinyl- oder lsochinolinylgruppe in der eine bis drei Methingruppen durch Stickstoffatome ersetzt sind, zu verstehen ist,
und die vorstehend erwähnten Heteroarylgruppen durch Fluor- und Chloratome ein- bis fünffach substituiert und R_{d} mono-, di- oder trisubstituiert sein können, wobei die Substituenten gleich oder verschieden sein können und R_{d} wie vorstehend erwähnt definiert ist,
unter den bei der Definition der vorstehend erwähnten Cycloalkylgruppen sowohl mono- als auch polycyclische Ringsysteme, die entweder verbrückt, spiro-verknüpft oder anelliert aufgebaut sind, zu verstehen sind,
unter den bei der Definition der vorstehend erwähnten Cycloalkenylgruppen sowohl mono- als auch polycyclische Ringsysteme, die entweder verbrückt oder anelliert aufgebaut sind, und die mindestens eine C=C-Doppelbindung tragen, zu verstehen sind,
wobei, soweit nichts anderes erwähnt wurde, die vorstehend erwähnten Alkyl-, Alkenyl- und Alkinylgruppen geradkettig oder verzweigt sein können,
wobei die Verbindung, in der R¹ ein Bromatom, R² eine 2-Butinylgruppe, X ein Sauerstoffatom und R³ eine Methylgruppe bedeuten, ausgeschlossen ist,
deren Tautomere, deren Enantiomere, deren Diastereomere, deren Gemische und deren Salze.

Des Weiteren schließen die in den vor- und nachstehenden Definitionen erwähnten gesättigten Alkyl- und Alkyloxyteile, die mehr als 2 Kohlenstoffatome enthalten, soweit nichts anderes erwähnt wurde, auch deren verzweigte Isomere wie beispielsweise die Isopropyl-, tert.Butyl-, Isobutylgruppe etc. ein.

Bevorzugt sind diejenigen Verbindungen der allgemeinen Formel I, in denen
R² und R³ wie oben erwähnt definiert sind,
R¹ ein Chlor- oder Bromatom und
X ein Sauerstoffatom oder eine -NH- oder -N(C₁₋₃-Alkyl)- Gruppe bedeuten,
wobei die Verbindung, in der R¹ ein Bromatom, R² eine 2-Butinylgruppe, X ein Sauerstoffatom und R³ eine Methylgruppe bedeuten, ausgeschlossen ist,
deren Tautomere, deren Enantiomere, deren Diastereomere, deren Gemische und deren Salze.
Besonders bevorzugt sind diejenigen Verbindungen der allgemeinen Formel I, in denen
R¹ ein Chlor- oder Bromatom,
R² eine durch eine Gruppe Rₐ substituierte C₁₋₃-Alkylgruppe, wobei Rₐ wie oben erwähnt definiert ist,
eine C₃₋₈-Alkenylgruppe,
eine durch ein Fluor-, Chlor- oder Bromatom oder durch eine Trifluormethylgruppe substituierte C₃₋₆-Alkenylgruppe oder
eine C₃₋₈-Alkinylgruppe,
X ein Sauerstoffatom und
R³ ein Wasserstoffatom,
eine C₁₋₂₀-Alkylgruppe, in der die Wasserstoffatome ganz oder teilweise durch Fluoratome ersetzt sein können und in der 1 bis 6 Methylengruppen jeweils durch ein Sauerstoff- oder Schwefelatom, durch eine -NH- oder -N(C₁₋₃-Alkyl)- Gruppe oder durch eine Carbonyl-, Sulfinyl- oder Sulfonylgruppe ersetzt sein können,
eine durch eine Gruppe R_{c} substituierte C₁₋₁₂-Alkylgruppe, in der die Wasserstoffatome ganz oder teilweise durch Fluoratome ersetzt sein können und in der 1 bis 4 Methylengruppen jeweils durch ein Sauerstoff- oder Schwefelatom, durch eine -NH-oder -N(C₁₋₃-Alkyl)- Gruppe oder durch eine Carbonyl-, Sulfinyl- oder Sulfonylgruppe ersetzt sein können, und wobei R_{c} wie oben erwähnt definiert ist,
eine C₃₋₈-Cycloalkylgruppe, in der die Wasserstoffatome ganz oder teilweise durch Fluoratome ersetzt sein können und in der 1 bis 4 Methylengruppen jeweils durch ein Sauerstoff- oder Schwefelatom, durch eine -NH- oder -N(C₁₋₃-Alkyl)- Gruppe oder durch eine Carbonyl-, Sulfinyl- oder Sulfonylgruppe ersetzt sein können,
eine C₃₋₂₀-Alkenylgruppe, in der die Wasserstoffatome ganz oder teilweise durch Fluoratome ersetzt sein können und in der 1 bis 6 Methylengruppen jeweils durch ein Sauerstoff- oder Schwefelatom, durch eine -NH- oder -N(C₁₋₃-Alkyl)- Gruppe oder durch eine Carbonyl-, Sulfinyl- oder Sulfonylgruppe ersetzt sein können,
eine C₃₋₂₀-Alkinylgruppe, in der die Wasserstoffatome ganz oder teilweise durch Fluoratome ersetzt sein können und in der 1 bis 6 Methylengruppen jeweils durch ein Sauerstoff- oder Schwefelatom, durch eine -NH- oder -N(C₁₋₃-Alkyl)- Gruppe oder durch eine Carbonyl-, Sulfinyl- oder Sulfonylgruppe ersetzt sein können, oder
eine Aryl-C₂₋₆-alkenylgruppe bedeuten,
wobei die Verbindung, in der R¹ ein Bromatom, R² eine 2-Butinylgruppe, X ein Sauerstoffatom und R³ eine Methylgruppe bedeuten, ausgeschlossen ist,
deren Tautomere, deren Enantiomere, deren Diastereomere, deren Gemische und deren Salze.
Ganz besonders bevorzugt sind diejenigen Verbindungen der allgemeinen Formel I, in denen
R¹ ein Chlor- oder Bromatom,
R² eine Phenylmethylgruppe, die am Phenylring durch ein Fluor-, Chlor-, Brom- oder lodatom oder durch eine Cyano- oder Methoxygruppe substituiert sein kann,
eine C₃₋₈-Alkenylgruppe,
eine C₃₋₈-Cycloalkenylmethylgruppe,
eine durch ein Fluor-, Chlor- oder Bromatom oder durch eine Trifluormethylgruppe substituierte C₃₋₆-Alkenylgruppe oder
eine C₃₋₈-Alkinylgruppe,
X ein Sauerstoffatom und
R³ ein Wasserstoffatom,
eine C₁₋₂₀-Alkylgruppe, in der die Wasserstoffatome ganz oder teilweise durch Fluoratome ersetzt sein können und in der 1 bis 6 Methylengruppen jeweils durch ein Sauerstoff- oder Schwefelatom oder durch eine -NH- oder -N(C₁₋₃-Alkyl)- Gruppen ersetzt sein können,
eine durch eine Gruppe R_{c} substituierte C₁₋₁₂-Alkylgruppe, in der die Wasserstoffatome ganz oder teilweise durch Fluoratome ersetzt sein können und in der 1 bis 4 Methylengruppen jeweils durch ein Sauerstoff- oder Schwefelatom oder durch eine -NH- oder -N(C₁₋₃-Alkyl)- Gruppe ersetzt sein können, und wobei R_{c} wie oben erwähnt definiert ist,
eine C₃₋₈-Cycloalkylgruppe, in der die Wasserstoffatome ganz oder teilweise durch Fluoratome ersetzt sein können und in der 1 bis 4 Methylengruppen jeweils durch ein Sauerstoff- oder Schwefelatom oder durch eine -NH- oder -N(C₁₋₃-Alkyl)- Gruppe ersetzt sein können,
eine C₃₋₂₀-Alkenylgruppe oder
eine C₃₋₂₀-Alkinylgruppe bedeuten,
wobei die Verbindung, in der R¹ ein Bromatom, R² eine 2-Butinylgruppe, X ein Sauerstoffatom und R³ eine Methylgruppe bedeuten, ausgeschlossen ist,
deren Tautomere, deren Enantiomere, deren Diastereomere, deren Gemische und deren Salze.
insbesondere jedoch diejenigen Verbindungen der allgemeinen Formel 1, in denen R¹ ein Bromatom,
R² eine 2-Buten-1-yl- oder 3-Methyl-2-buten-1-yl-Gruppe,
eine 2-Butin-1-yl-Gruppe oder
eine 2-Chlorphenylmethyl- oder 2-Bromphenylmethyl-Gruppe,
X ein Sauerstoffatom, und
R³ eine C₁₋₁₀-Alkylgruppe oder C₃₋₈-Cycloalkylgruppe bedeuten,
wobei die Verbindung, in der R¹ ein Bromatom, R² eine 2-Butinylgruppe, X ein Sauerstoffatom und R³ eine Methylgruppe bedeuten, ausgeschlossen ist,
deren Tautomere, deren Enantiomere, deren Diastereomere, deren Gemische und deren Salze.

Folgende Verbindungen der allgemeinen Formel I sind besonders bevorzugt:
(a) 2-Brom-5-formyl-3-(3-methyl-2-buten-1-yl)-3H-imidazol-4-carbonsäuremethylester und
(b) 2-Brom-5-formyl-3-(2-butin-1-yl)-3H-imidazol-4-carbonsäureethylester
sowie deren Salze.

Erfindungsgemäß erhält man die Verbindungen der allgemeinen Formel I nach an sich bekannten Verfahren, beispielsweise nach folgenden Verfahren:
a) Reduktion einer Verbindung der allgemeinen Formel 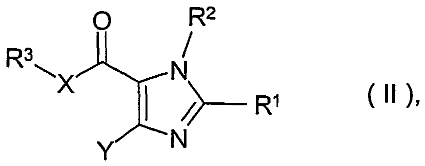 in der

R¹ bis R³ und X wie eingangs erwähnt definiert sind und Y eine Carbonsäure-, Carbonsäureamid-, Nitril-, Carbonsäureester-, Carbonsäurethioester-, Carbonsäureanhydrid- oder Carbonsäurechlorid-Gruppe, die jeweils über das Carboxylkohlenstoffatom an den Imidazolring gebunden sind, bedeutet.

Reduktionen von den genannten Carbonsäurederivaten zu Aldehyden sind Standardtransformationen in der synthetischen Organischen Chemie. Eine Übersicht der verschieden Möglichkeiten findet sich in Steven D. Burke, Rick L. Danheiser, Oxidizing and Reducing Agents, Weinheim: John Wiley, 1999 sowie in J. Seyden-Penne, Reductions by the Alumino- and Borohydrides in Organic Synthesis, Weinheim: VCH, 1991.

### Stellvertretend sind nachfolgend einige Beispiele genannt:

Carbonsäuren lassen sich beispielsweise mit Alkalimetallen wie Lithium in Ammoniak oder Methylaminen und anschließender Hydrolyse des entstehenden Imins in Aldehyde überführen. Des Weiteren kommen Metallhydride wie Diisobutylaluminiumhydrid oder Borane wie Thexylchlor- oder -bromboran dafür in Frage. Die Reaktionen können bei Temperaturen zwischen -80°C und 100°C durchgeführt werden, vorzugsweise zwischen -70°C und 40°C.

Carbonsäureamide oder Nitrile lassen sich z.B. mit Diisobutylaluminiumhydrid, Lithium- oder Natriumaluminiumhydrid, Lithiumtri-tert.-butoxyaluminiumhydrid und Diaminoaluminiumhydriden in Lösungsmitteln wie Tetrahydrofuran, Ether, Toluol, Hexan oder Dichlormethan zu den entsprechenden Aldehyden umsetzen. Die Reaktionen können bei Temperaturen zwischen -80°C und 100°C durchgeführt werden, bevorzugt zwischen -70°C und 25°C.

Unter den Beispielen für Reduktionsmittel, die Carbonsäureester oder -anhydride zu Aldehyden umformen können, sind Hydride wie Diisobutylaluminiumhydrid, Diaminoaluminiumhydride, Lithiumtri-tert.-butoxyaluminiumhydrid und Natrium-bis(2-methoxy-ethoxy)aluminiumhydrid in Lösungsmitteln wie Dichlormethan, Toluol oder Hexan als besonders geeignet zu nennen. Die Reaktionen können bei Temperaturen zwischen -80°C und 100°C durchgeführt werden, bevorzugt zwischen -70°C und 0°C.

Carbonsäurethioester lassen sich beispielsweise durch Übergangsmetalle bzw. Übergangsmetallkomplexe, wie z.B. Palladium oder Nickel, in Gegenwart von Hydriden wie Trialkylsilanen oder molekularem Wasserstoff in Aldehyde überführen. Die Anwendung von Metallhydriden wie Diisobutylaluminiumhydrid in Lösungsmitteln wie Hexan, Dichlormethan oder Toluol ist ebenfalls eine allgemein verbreitete Methode zur Reduktion von Thioestern zu Aldehyden. Die Reaktionen können bei Temperaturen zwischen -80°C und 100°C durchgeführt werden, bevorzugt zwischen -70°C und 25°C.

Carbonsäurechloride lassen sich u.a. mit Wasserstoff in Gegenwart von Übergangsmetallen, wie z.B. Palladium auf Kohle oder Bariumsulfat, zu den entsprechenden Aldehyden überführen. Natriumborhydrid in Dimethylformamid und Tetrahydrofuran oder Lithiumtri-tert.-butoxyaluminiumhydrid in Diglyme sind in vielen Fällen genauso geeignet. Die Reaktionen können bei Temperaturen zwischen -100°C und 100°C ausgeführt werden. In Gegenwart von Übergangsmetallkatalysatoren sind Temperaturen zwischen 0°C und 30°C bevorzugt, wogegen die Reaktionen mit Metallhydriden am besten zwischen -30°C und -80°C durchgeführt werden.
b) Oxidation einer Verbindung der allgemeinen Formel 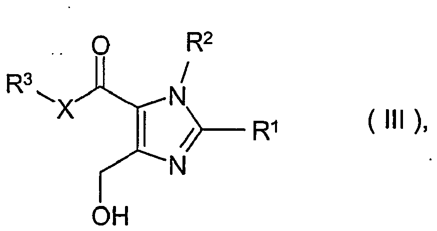 in der R¹, R², R³ und X wie eingangs erwähnt definiert sind.

Die Oxidation einer Hydroxymethylgruppe zu einem Aldehyd ist eine Standardreaktion in der synthetischen Organischen Chemie. Als Oxidationsmittel.kommen eine Vielzahl von Reagenzien in Frage. Eine Übersicht über Oxidationsmethoden bzw. -reagenzien zur Umformung von Alkoholen zu Aldehyden findet sich in Steven D. Burke, Rick L. Danheiser, Oxidizing and Reducing Agents, Weinheim: John Wiley, 1999 sowie in Milos Hudlicky, Oxidation in Organic Chemistry, Washington: ACS, 1990.

### Als Beispiele seien genannt:

Oxidationen mit Dimethylsulfoxid in Gegenwart von beispielsweise Oxalylchlorid, Acetanhydrid oder Schwefeltrioxid-Pyridin-Komplex. Eine sehr milde Oxidation ist mit Verbindungen von Iod in hohen Oxidationsstufen wie im sogenannten Dess-Martin-Periodinan durchführbar. Oxidationen durch Dehydrierung mittels eines Übergangsmetalls bzw. Übergangsmetallsalzes, wie z.B. Kupferoxid, Palladiumacetat oder Raney-Nickel, sind ebenfalls möglich. N-lod-, N-Brom- oder N-Chlorsuccinimid sind ebenfalls geeignete Oxidationsmittel für die genannte Transformation. Oxidationen mit Metallen in hohen Oxidationsstufen bzw. deren Komplexe und Salze, wie z.B. Braunstein, Bariummanganat, Pyridiniumchlorochromat, Pyridiniumdichromat, Kalium- oder Natriumdichromat, Cerammoniumnitrat, Silbercarbonat, Bleitetraacetat oder Tetrapropylammoniumperrhutenat sind auch breit anwendbar. Die Metalle können dabei in stöchiometrischer Menge oder, in Gegenwart eines geeigneten Co-Oxidans, wie beispielsweise N-Methylmorpholin-N-oxid, Natriumhypochlorid oder Natriumbromat, in katalytischen Mengen eingesetzt werden. Die Lösungsmittelwahl ist vom Reagenz abhängig, jedoch kommen in vielen Fällen Tetrahydrofuran, Ether, Dioxan, Toluol, Hexan, Dichlormethan, Essigsäureethylester, Sulfolan oder Dimethylformamid in Frage. Die Reaktionen können bei Temperaturen zwischen -80°C und 100°C durchgeführt werden. Bevorzugt werden die Umsetzungen zwischen 0°C und 60°C durchgeführt.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-O-p-toluoyl-weinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Des Weiteren können die erhaltenen Verbindungen der Formel I in ihre Salze übergeführt werden. Als Säuren kommen hierfür beispielsweise Salzsäure, Bromwasserstoffsäure, Schwefelsäure, Methansulfonsäure, Phosphorsäure oder Ameisensäure in Betracht.

Außerdem lassen sich die so erhaltenen neuen Verbindungen der Formel I, falls diese eine Carboxygruppe enthalten, gewünschtenfalls anschließend in ihre Salze mit anorganischen oder organischen Basen überführen. Als Basen kommen hierbei beispielsweise Natriumhydroxid, Kaliumhydroxid, Arginin, Cyclohexylamin, Ethanolamin, Diethanolamin und Triethanolamin in Betracht.

Die als Ausgangsstoffe verwendeten Verbindungen der allgemeinen Formeln II und III sind entweder literaturbekannt oder man erhält diese nach an sich literaturbekannten Verfahren (siehe Beispiele 1 und 2).

Ein weiterer Erfindungsgegenstand ist neben dem Herstellungsverfahren für die Verbindungen der allgemeinen Formel I auch die Verwendung der Verbindurigen der allgemeinen Formel I zur Synthese von Imidazo[4,5-d]pyridazin-4-onen der allgemeinen Formel IV gemäß Schema 1 sowie das Gesamtverfahren zur Herstellung von lmidazo[4,5-d]pyridazin-4-onen der allgemeinen Formel IV ausgehend von kommerziell erhältlichen Imidazol-Derivaten. Der hier beschriebene Zugang zu Verbindungen der allgemeinen Formel I in Kombination mit deren weiterer Umsetzung mit Hydrazinen ergibt einen äußerst effizienten und variablen Syntheseweg zu Imidazo[4,5-d]pyridazin-4-onen des Typs V (Schema 2), die sehr gute Inhibitoren der Dipeptidylpeptidase IV (DPP IV) sind und zur Behandlung von Diabetes eingesetzt werden können. Die dargestellte Vorgehensweise lässt eine breite und effiziente Variation der Substituenten R¹, R² und R⁴ der Imidazo[4,5-d]pyridazin-4-one zu, die eine schnelle Optimierung aller Substituenten bezüglich erwünschter und unerwünschter biologischer Wirkung ermöglichen. Die Möglichkeit der Variation von R¹ und R⁴ auf einer späten Stufe der Synthesesequenz stellt einen deutlichen Vorteil gegenüber dem in WO 03/104229 dargestellten Vorgehen dar, nach dem nur an R⁴ auf einer späten Stufe der Synthese variiert werden kann. Die Synthese eines bestimmten lmidazo[4,5-d]pyridazin-4-ons, wie z.B. von dem in Schema 2 dargestellten, ist auf dem vorgestellten Weg in nur vier bzw. fünf Synthesestufen ausgehend vom literatur-bekannten 2-Brom-1*H*-imidazol-4,5-dicarbonsäuredimethylester möglich. Auch diesbezüglich stellt der dargestellte Syntheseweg eine deutliche Verbesserung gegenüber dem in WO 03/104229 dargestellten Syntheseweg dar, der die gleiche Verbindung in neun bzw. zehn Reaktionsschritten ausgehend vom literatur-bekannten 2-Brom-1*H*-imidazol-4,5-dicarbonsäurenitril zugänglich machen würde.

Schema 1: Synthese von Imidazo[4,5-d]pyridazin-4-onen (IV) ausgehend von Verbindungen der allgemeinen Formel I

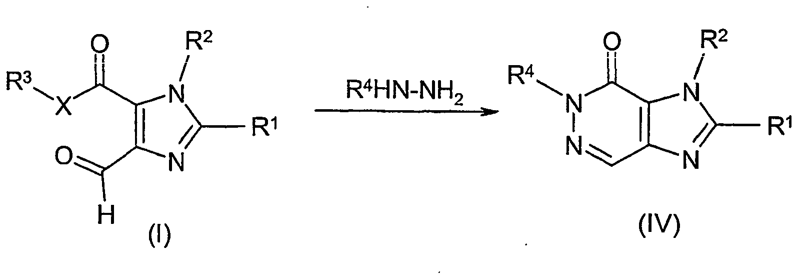

Schema 1 zeigt ein Verfahren zur Herstellung von Imidazo[4,5-d]pyridazin-4-onen der allgemeinen Formel IV, in der R¹ und R² wie eingangs erwähnt definiert sind, ausgehend von der Verbindung der allgemeinen Formel I, in der R¹ bis R³ und X wie eingangs erwähnt definiert sind, und eines durch R⁴ substituierten Hydrazins, wobei
R⁴ beispielsweise ein Wasserstoffatom,
eine C₃₋₈-Alkylgruppe,
eine durch eine Gruppe Rₐ substituierte C₁₋₃-Alkylgruppe, wobei Rₐ wie oben erwähnt definiert ist,
eine C₃₋₈-Alkenyl- oder C₃₋₈-Alkinylgruppe,
eine Arylcarbonylmethyl- oder Heteroarylcarbonylmethylgruppe,
eine Arylprop-2-enyl- oder Heteroarylprop-2-enylgruppe oder
eine Arylgruppe oder Heteroarylgruppe bedeuten kann.

Die Verbindungen der Formel IV können durch Umsetzung von Verbindung I mit einem durch R⁴ substituierten Hydrazin oder einem geschützten Hydrazinderivat davon, wie z.B. 1,2-Bis-(t-butyldimethylsilyl)hydrazin, in einem Lösungsmittel wie z.B. Wasser, Alkohol, wie Methanol, Ethanol, Propanol, Isopropanol, Butanol, Ether, Dioxan, Tetrahydrofuran, Dimethylsulfoxid, Dimethylformamid, Dimethylacetamid, N-Methylpyrrolidon, Ethylacetat, Benzol oder Toluol synthetisiert werden. Die Zugabe von Additiven wie Säuren oder Basen kann die Reaktion in Abhängigkeit von X fördern. Als organische Säuren eignen sich z.B. Toluolsulfonsäure, Ameisensäure, Essigsäure, Oxalsäure oder Zitronensäure. Als Beispiele für anwendbare anorganische Säuren sind Salzsäure, Borsäure oder Schwefelsäure zu nennen. Saure Tonerden wie z.B. Montmorillonite oder Lewis-Säuren wie beispielsweise Eisenchlorid, Lithiumchlorid, Lithiumperchlorat oder Magnesiumchlorid können ebenfalls Anwendung finden. Geeignete anorganische Basen sind z.B. Lithiumhydroxid, Natriumhydroxid, Kaliumhydroxid, Natriumcarbonat, Kaliumcarbonat, Natriumhydrogencarbonat, Aluminiumoxid oder Natriumacetat. Organische Basen wie z.B. Triethylamin, Diisopropylethylamin, Diazabicycloundekan, Diazabicyclononan, Diazabicyclooktan, Pyridin, Dimethylaminopyridin, N-Methylpiperidin oder Hexamethyldisilazan können ebenfalls geeignet sein. Auch Umsetzungen im entsprechenden Additiv ohne zusätzliches Lösungsmittel sind möglich. Die Reaktionen können bei Temperaturen zwischen -80°C und 200°C durchgeführt werden, bevorzugt zwischen 0°C und 120°C. Die Reaktionen können auch unter Mikrowellenbestrahlung durchgeführt werden.

Die danach erhaltenen Imidazo[4,5-d]pyridazin-4-one lassen sich durch geeignete Substitution nach an sich bekannten Verfahren beispielsweise in gute Inhibitoren der Dipeptidylpeptidase IV (DPP IV) umformen, die wiederum Anwendung in der Behandlung von Diabetes finden können. Schema 2 gibt anhand eines Beispieles die Synthese einer solchen Verbindung ausgehend von einer Verbindung der allgemeinen Formel I wieder.

Verbindungen der allgemeinen Formel I können zur effizienten Synthese von lmidazo[4,5-d]pyridazin-4-onen der allgemeinen Formel IV verwendet werden. Durch geeignete Substitution lassen sich daraus gute DPPIV-Inhibitoren gewinnen, wie anhand des Beispiels in Schema 2 wiedergegeben ist.

### Schema 2: Synthese eines DPP IV-Inhibitors ausgehend von einer Verbindung der allgemeinen Formel I

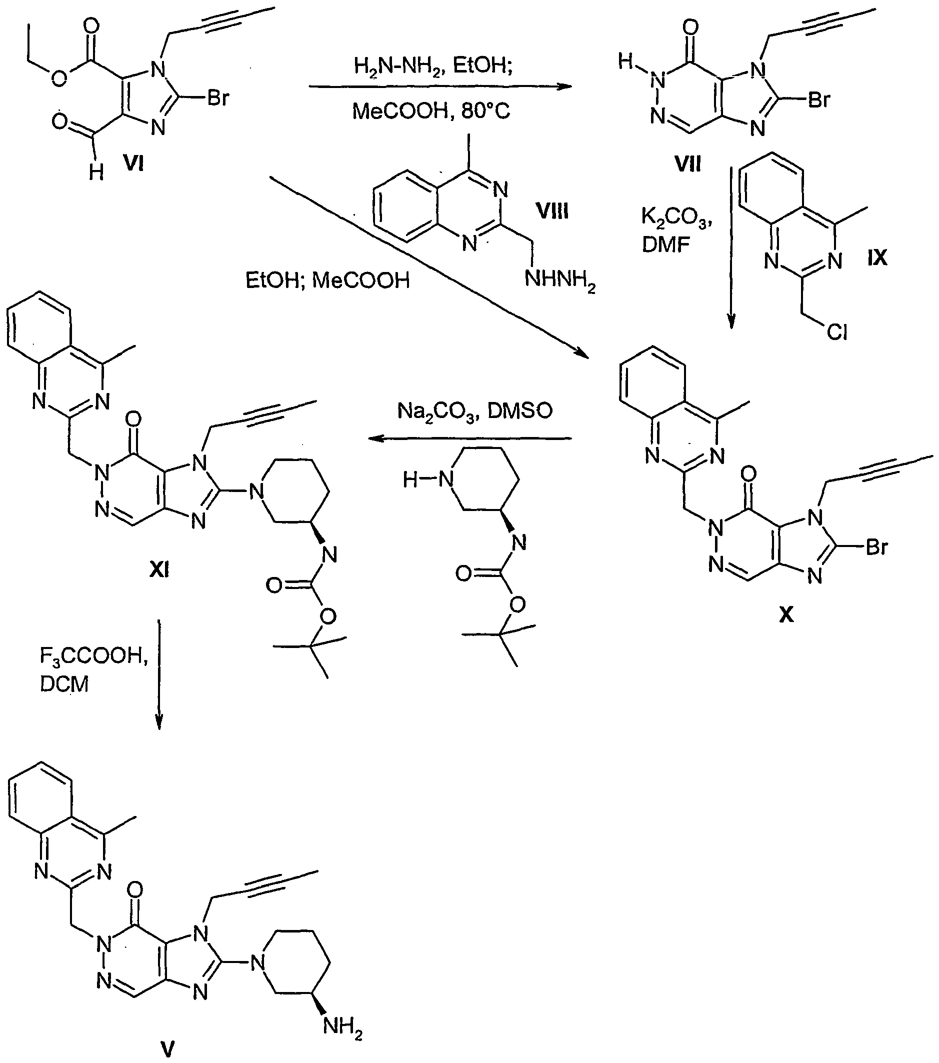

Die Synthesesequenz beginnt mit der Umsetzung von VI, das ein konkretes Beispiel für Verbindungen der allgemeinen Formel I darstellt, mit Hydrazinhydrat in Ethanol. Nach vollständiger Umsetzung von VI mit Hydrazin zum Hydrazon, wird Essigsäure zugegeben und die Lösung bei 100°C gerührt. Danach wird VII in hoher Ausbeute erhalten. Alkylierung von VII mit 2-(Chlormethyl)-4-methylchinazolin IX in Gegenwart von Kaliumcarbonat in DMF ergibt X. Alternativ kann X auch direkt aus VI mit 2-(Hydrazinylmethyl)-4-methyl-chinazolin VIII synthetisiert werden. Dazu wird wie von VI zu VII vorgegangen. X wird dann mit (*R*)-3-t-Butyloxycarbonylamino-piperidin in DMSO bei 70°C zu XI umgesetzt, das danach in guter Ausbeute erhalten wird. Im abschließenden Reaktionsschritt wird die Schutzgruppe in XI, t-Butyloxycarbonyl, unter sauren Bedingungen, z.B. mit Trifluoressigsäure oder alkoholischer Salzsäure in Dichlormethan, abgespalten. Der DPP IV-Inhibitor V wird so in nur drei bzw. vier Reaktionsschritten in durchweg hohen Ausbeuten erhalten.

Die nachfolgenden Beispiele sollen die Erfindung näher erläutern:

### Beispiel 1

### 2-Brom-3-(3-methyl-2-buten-1-yl)-5-formyl-3H-imidazol-4-carbonsäure-methylester

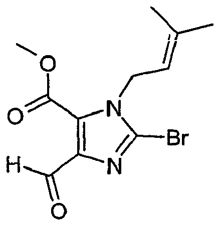

### 1a) 2-Brom-1H-imidazol-4,5-dicarbonsäuredimethylester

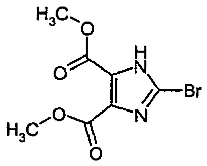

Bei Raumtemperatur wurden zu einer Lösung von 9.90 g (53,76 mmol) Imidazol-4,5-dicarbonsäuredimethylester in 300 ml Dichlormethan und 80 ml Acetonitril 9,50 g (59,45 mmol) Brom getropft. Es wurden 7,46 g (54,00 mmol) Kaliumcarbonat zugegeben, und das Reaktionsgemisch wurde 2 Stunden bei Raumtemperatur gerührt. Danach wurde das Dichlormethan am Rotationsverdampfer entfernt und der Rückstand mit einer gesättigten Lösung von Natriumchlorid und Natriumthiosulfat versetzt. Es wurde zehnmal mit Ethylacetat extrahiert, die organischen Extrakte wurden über Natriumsulfat getrocknet und dann vom Lösungsmittel befreit.
Ausbeute: 12,31 g (87% der Theorie)
C₇H₇BrN₂O₄ (263,05)
Massenspektrum: (M+H)⁺ = 263/265 (Brom)

### 1b) 2-Brom-1-(3-methyl-2-buten-1-yl)-1H-imidazol-4,5-dicarbonsäuredimethylester

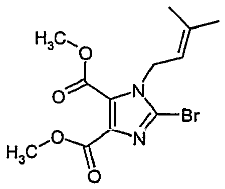

Eine Mischung von 12,20 g (46 mmol) 2-Brom-imidazol-4,5-dicarbonsäuredimethyl-ester, 7,16 g (48 mmol) 1-Brom-3-methyl-2-buten und 7,19 g (52 mmol) Kaliumcarbonat in 150 ml Dimethylformamid wurden zwei Stunden bei 50°C gerührt. Dann wurden 100 ml Wasser zugegeben, und es wurde drei Mal mit Ethylacetat extrahiert. Die Extrakte wurden getrocknet und eingeengt. Das so erhaltene Rohprodukt wurde durch Säulenchromatographie gereinigt (Kieselgel; Elutionsmittel: Cyclohexan/Ethylacetat 4:1->2:1).
Ausbeute: 13,52 g (89% der Theorie)
C₁₂H₁₅BrN₂O₄ (331,16)
Massenspektrum: (M+H)⁺ = 331/333 (Brom)

### 1c) 2-Brom-3-(3-methyl-2-buten-1-yl)-5-formyl-3H-imidazol-4-carbonsäuremethylester

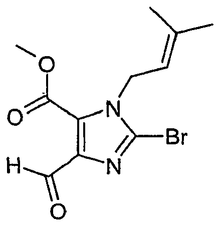

Unter Argon-Atmosphäre wurden bei -65°C zu einer Lösung von 9,00 g (12,18 mmol) 2-Brom-1-(3-methyl-2-buten-1-yl)-1*H*-imidazol-4,5-dicarbonsäuredimethylester in 140 ml Tetrahydrofuran 35 ml (35 mmol) Diisobutylaluminiumhydrid (1 mol/l in Toluol) innerhalb von 20 Minuten getropft. Nach 1 Stunde bei -65°C wurden noch einmal 5 ml (5 mmol) Diisobutylaluminiumhydrid (1 mol/l in Toluol) zugetropft. Es wurde noch eine weitere Stunde bei -65°C gerührt und dann 20 ml einer 1:1-Mischung aus Salzsäure (1 mol/l) und Tetrahydrofuran zugetropft. Nach dem Erwärmen auf Raumtemperatur wurden ca. 100 ml Wasser zugegeben, und es wurde drei Mal mit je 70 ml Essigester extrahiert. Die Extrakte wurden über Natriumsulfat getrocknet, eingeengt, und das so erhaltene Rohprodukt wurde durch Säulenchromatographie gereinigt (Kieselgel; Elutionsmittel: Cyclohexan/Essigsäureethylester 3:1->1:2).
Ausbeute: 7,03 g (86% der Theorie)
C₁₁H₁₃BrN₂O₃ (301,14)
Massenspektrum: (M+H)⁺ = 301/303 (Brom)

### Beispiel 2

### 2-Brom-3-(but-2-inyl)-5-formyl-3H-imidazol-4-carbonsäureethylester

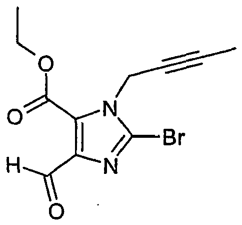

### 2a) 2-Brom-1H-imidazol-4,5-dicarbonsäurediethylester

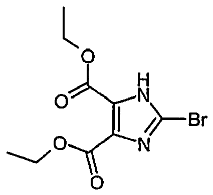

Unter Argonatmosphäre wurden zu einer Lösung von 2,60 g (12,25 mmol) Imidazol-4,5-dicarbonsäurediethylester in 30 ml Acetonitril 3,30 g (18,54 mmol) N-Bromsuccinimid gegeben. Die Lösung wurde 24 Stunden im Dunkeln bei Raumtemperatur gerührt und dann eingeengt. Der Rückstand wurde in 150 ml Ethylacetat aufgenommen und jeweils zwei Mal mit gesättigter wässriger Natriumchloridlösung und gesättigter wässriger Natriumthiosulfat und noch einmal mit gesättigter wässriger Natriumchloridlösung gewaschen. Die organische Phase wurde über Natriumsulfat getrocknet und das Lösungsmittel entfernt.
Ausbeute: 3,50 g (98% der Theorie)
C₉H₁₁BrN₂O₄ (291,10)
Rf-Wert: 0.6 (Kieselgel; Dichlormethan/Ethanol 9:1)
Massenspektrum: (M+H)⁺ = 291/293 (Brom)

### 2b) 2-Brom-1-(but-2-inyl)-1H-imidazol-4,5-dicarbonsäurediethylester

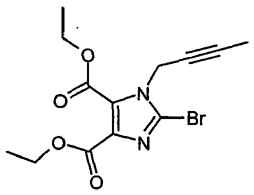

Hergestellt analog **1b** aus 3,50 g (12,02 mmol) 2-Brom-imidazol-4,5-dicarbonsäure-diethylester mit 1,09 ml (12,1 mmol) 1-Brom-2-butin und 2,07 g (15,00 mmol) Kaliumcarbonat in 15 ml Dimethylformamid.
Ausbeute: 3,60 g (87% der Theorie)
C₁₃H₁₅BrN₂O₄ (343,17)
Rf-Wert: 0.8 (Kieselgel; Dichlormethan/Ethanol 9:1)
Massenspektrum: (M+H)⁺ = 343/345 (Brom)

### 2c) 2-Brom-3-(but-2-inyl)-5-formyl-3H-imidazol-4-carbonsäureethylester

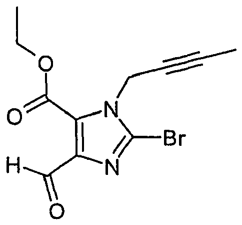

Hergestellt analog **1c** aus 20,00 g (58,28 mmol) 2-Brom-1-(but-2-inyl)-1H-imidazol-4,5-dicarbonsäurediethylester mit 70 ml (70 mmol) Diisobutylaluminiumhydrid in 300 ml Tetrahydrofuran.
Ausbeute: 15,90 g (91 % der Theorie)
C₁₁H₁₁BrN₂O₃ (299,12)
Massenspektrum: (M+H)⁺ = 299/301 (Brom)

Analog den vorstehend genannten Beispielen und anderen literaturbekannten Verfahren können die folgenden Verbindungen hergestellt werden:

| Bsp. | Struktur | Bsp. | Struktur | Bsp. | Struktur |
|---|---|---|---|---|---|
| (1) | 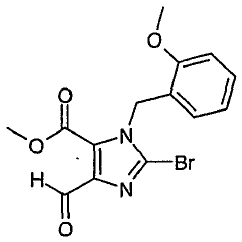 | (2) | 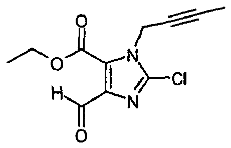 | (3) | 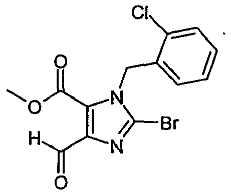 |
| (4) | 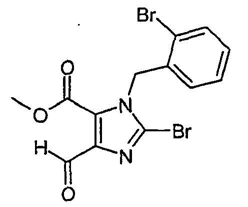 | (5) | 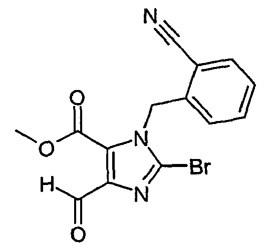 | (6) | 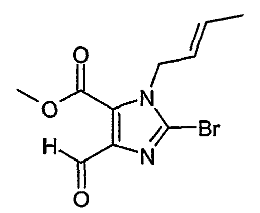 |
| (7) | 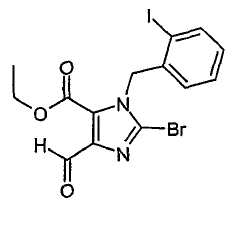 | (8) | 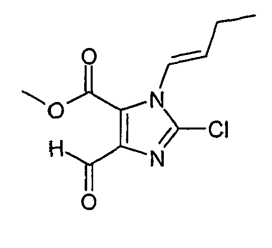 | (9) | 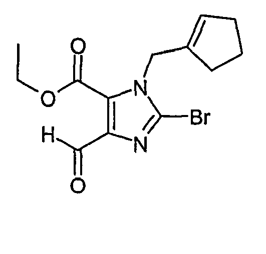 |
| (10) | 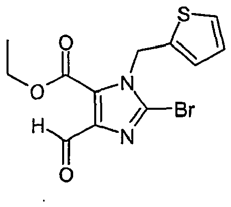 | (11) | 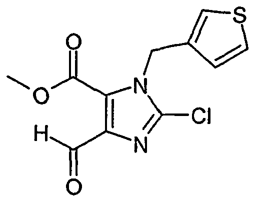 | (12) | 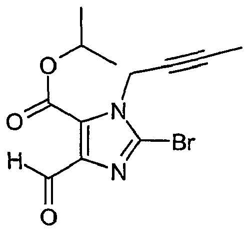 |
| (13) | 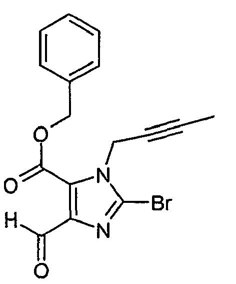 | (14) | 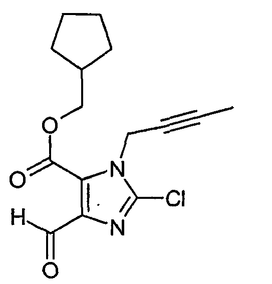 | (15) | 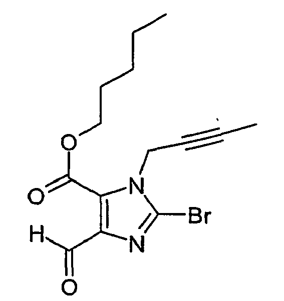 |
| (16) | 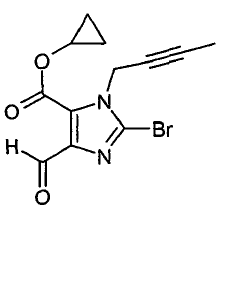 | (17) | 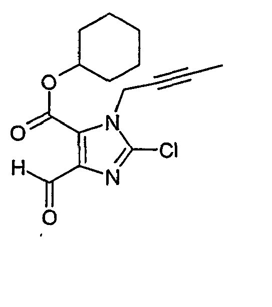 | (18) | 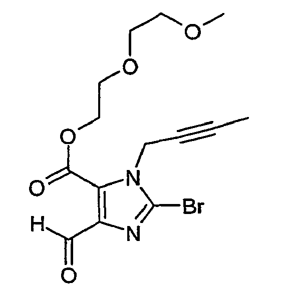 |
| (19) | 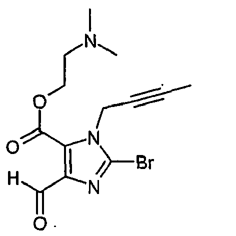 | (20) | 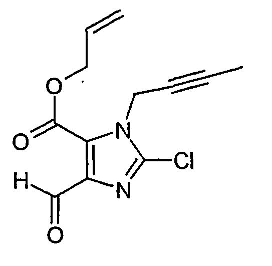 | (21) | 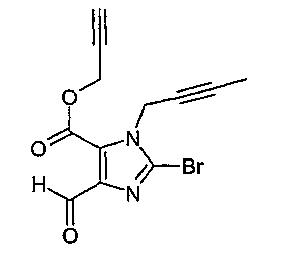 |
| (22) | 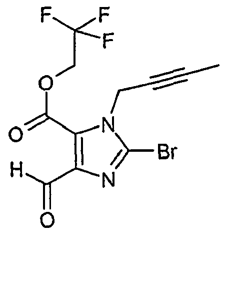 | (23) | 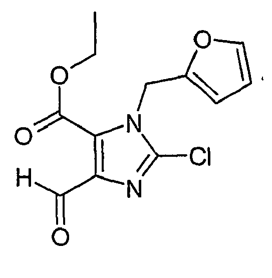 | (24) | 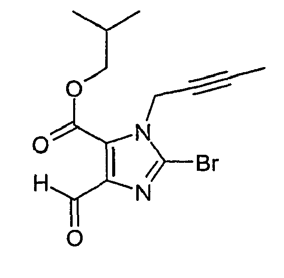 |
| (25) | 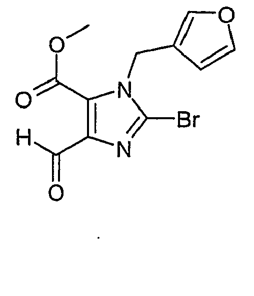 | (26) | 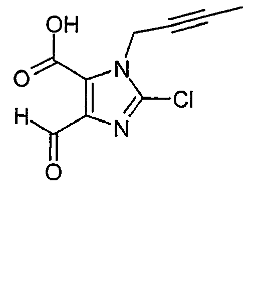 | (27) | 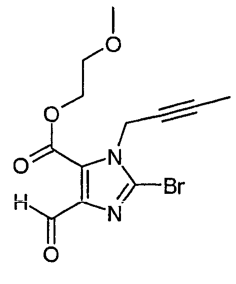 |
| (28) | 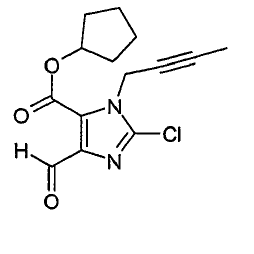 | (29) | 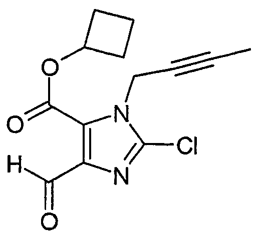 | (30) | 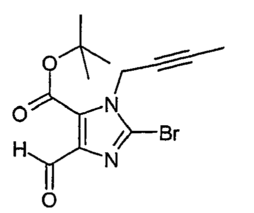 |
| (31) | 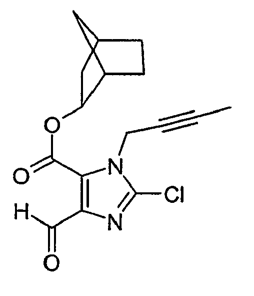 | (32) | 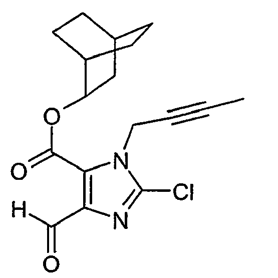 | (33) | 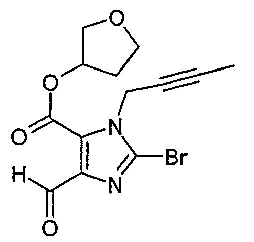 |
| (34) | 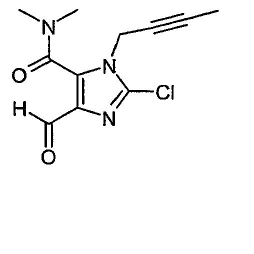 | (35) | 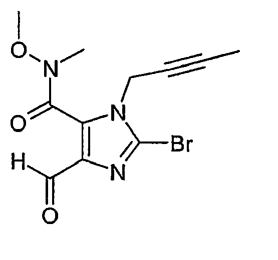 | (36) | 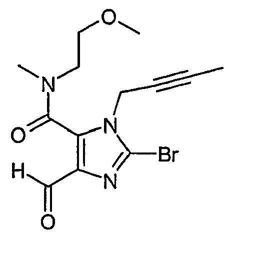 |

### Beispiel 3

### a) 2-Brom-3-(but-2-inyl)-3,5-dihydro-imidazo[4,5-d]pyridazin-4-on (VII)

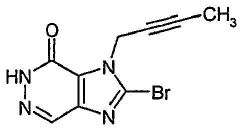

Zu einer Lösung von 35,0 g (0,117 mol) 2-Brom-3-(but-2-inyl)-5-formyl-3*H*-imidazol-4-carbonsäure-ethylester in 400 ml Ethanol wurden bei Raumtemperatur 6,31 ml (0,126 mol) Hydrazinhydrat getropft. Nach 30 min Rühren wurden 27 ml (0,472 mol) konzentrierte Essigsäure zugefügt und die Lösung anschließend 1 h bei 100°C gerührt. Nach dem Abkühlen wurde der ausgefallene Feststoff abgesaugt, mit Ethanol und Diethylether gewaschen und getrocknet. Der Feststoff wurde noch durch Umkristallisieren aus EtOH gereinigt.
Ausbeute: 84% der Theorie.
C₉H₇BrN₄O (267.09)
Massenspektrum: (M+H)⁺ = 267/269 (Br)

### b)2-Brom-3-(but-2-inyl)-5-(4-methyl-chinazolin-2-ylmethyl)-3,5-dihydro-imidazo[4,5-d]pyridazin-4-on (X)

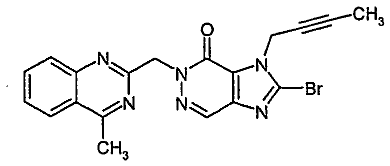

Zu einer Lösung von 300 mg (1,12 mmol) 2-Brom-3-(but-2-inyl)-3,5-dihydro-imidazo-[4,5-d]pyridazin-4-on und 344 mg (1,35 mmol) 2-Chlormethyl-4-methyl-chinazolin in 4 ml Dimethylformamid wurden 489 mg (1,5 m,ol) Cesiumcarbonat gegeben und dieses Gemisch 1 h unter Argon-Atmosphäre bei 80°C gerührt. Dann wurde mit 10 ml Wasser verdünnt, die Lösung auf ca. 10°C gekühlt, der ausgefallene Niederschlag abgesaugt und getrocknet und durch Säulenchromatographie gereinigt (Kieselgel; Elutionsmittel: Dichlormethan/Ethanol 1:0->19:1).
Ausbeute: 94% der Theorie.
C₁₉H₁₅BrN₆O (423.28)
Massenspektrum: (M+H)⁺ = 423/425 (Br)

### c) (R)-{1-[1-(But-2-inyl)-6-(4-methyl-chinazolin-2-ylmethyl)-7-oxo-6,7-dihydro-1H-imidazo[4,5-d]pyridazin-2-yl]-piperidin-3-yl}-carbaminsäure-tert-butylester (XI)

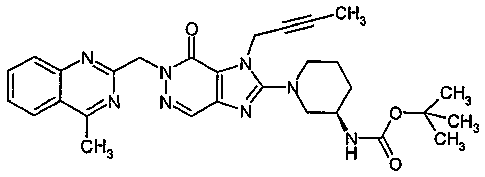

Eine Lösung von 240 mg (0,57 mmol) 2-Brom-3-(but-2-inyl)-5-(4-methyl-chinazolin-2-ylmethyl)-3,5-dihydro-imidazo[4,5-d]pyridazin-4-on und 140 mg (0,70 mmol) (R)-Piperidin-3-yl-carbaminsäure-tert-butylester in 4 ml Dimethylsulfoxid wurde mit 95 mg (0,90 mmol) Natriumcarbonat versetzt und 3 h bei 80°C gerührt. Dann wurden nochmals 50 mg Piperidin-3-yl-carbaminsäure-tert-butylester hinzugefügt und weitere 2 h bei 80°C gerührt. Nach Abkühlen auf Raumtemperatur wurde mit 10 ml Wasser versetzt und 30 Minuten gerührt. Das dabei ausgefallene Produkt wurde abgesaugt, mit 5 ml Wasser gewaschen und getrocknet.
Ausbeute: 81% der Theorie.
C₂₉H₃₄N₈O₃ (542.65)
Massenspektrum: (M+H)⁺ = 543

### d) (R)-2-(-3-Amino-piperidin-1-yl)-3-(but-2-inyl)-5-(4-methyl-chinazolin-2-ylmethyl)-3,5-dihydro-imidazo[4,5-d]pyridazin-4-on (V)

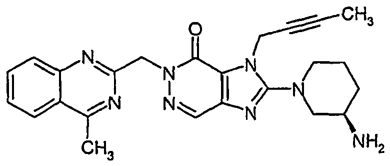

Eine Lösung von 17,2 g (31,7 mmol) (*R*)-{1-[1-(But-2-inyl)-6-(4-methyl-chinazolin-2-ylmethyl)-7-oxo-6,7-dihydro-1*H*-imidazo[4,5-d]pyridazin-2-yl]-piperidin-3-yl}-carbaminsäure-tert-butylester in 400 ml Dichlormethan wurde mit 85 ml Trifluoressigsäure versetzt und drei Stunden bei Raumtemperatur gerührt. Das Gemisch wurde bei 30°C zur Trockne eingedampft, der Rückstand in Dichlormethan gelöst und die Lösung mit eisgekühlter gesättigter Natriumcarbonat-Lösung basisch gestellt. Die organische Phase wurde abgetrennt und die wässrige noch zwei Mal mit Dichlormethan extrahiert. Die vereinten organischen Phasen wurden über Natriumsulfat getrocknet und das Lösungsmittel entfernt. Der Rückstand wurde mittels Chromatographie auf Kieselgel gereinigt (Dichlormethan/Methanol/Ammoniumhydroxid 95:5:0,1).
Ausbeute: 89% der Theorie.
C₂₄H₂₆N₈O(442.53)
Massenspektrum: (M+H)⁺ = 443

### Analog Beispiel 3 wurden folgende Verbindungen erhalten:

| Bsp. | Struktur | Bsp. | Struktur |
|---|---|---|---|
| (1) | 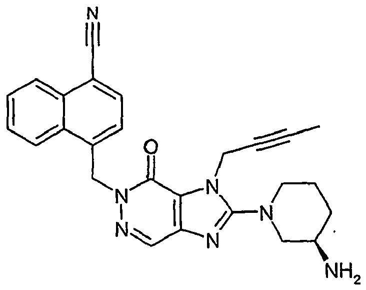 | (2) | 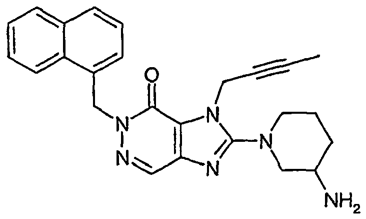 |
| | Massenspektrum: (M+H)⁺ = 452 | | Massenspektrum: (M+H)⁺ = 427 |
| (3) | 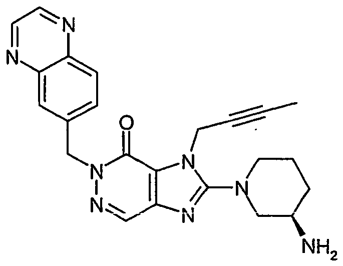 | (4) | 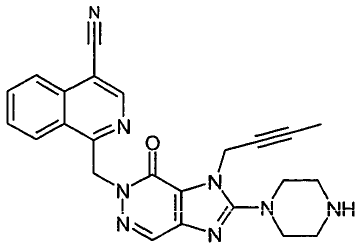 |
| | Massenspektrum: (M+H)⁺ = 429 | | Massenspektrum: (M+H)⁺ = 439 |
| (5) | 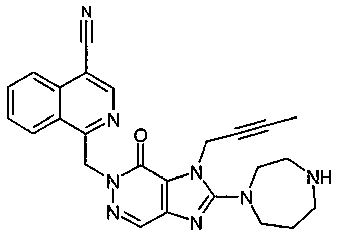 | (6) | 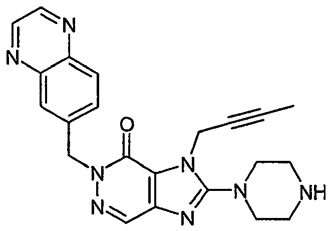 |
| | Massenspektrum: (M+H)⁺ = 453 | | Massenspektrum: (M+H)⁺ = 415 |
| (7) | 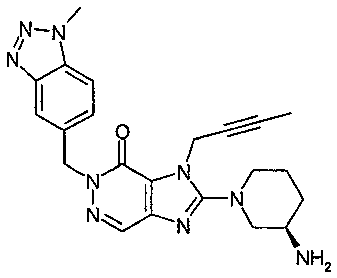 | (8) | 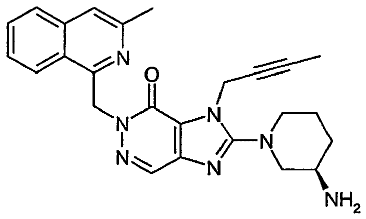 |
| | Massenspektrum: (M+H)⁺ = 432 | | Massenspektrum: (M⁺H)⁺ = 442 |
| (9) | 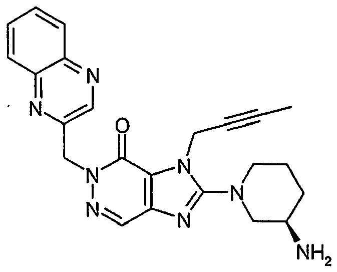 | (10) | 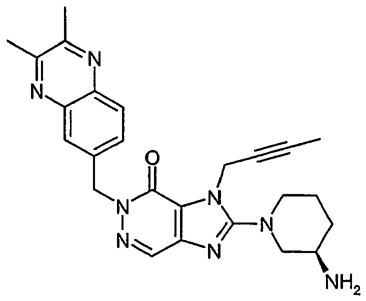 |
| | Massenspektrum: (M+H)⁺ = 429 | | Massenspektrum: (M+H)⁺ = 457 |
| (11) | 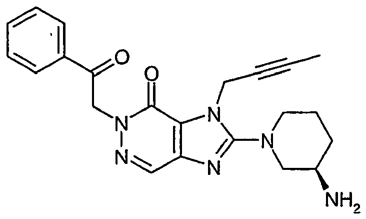 | (12) | 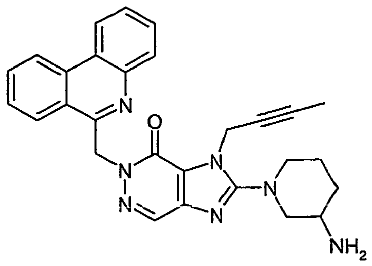 |
| | Massenspektrum: (M+H)⁺ = 405 | | Massenspektrum: (M+H)⁺ = 478 |
| (13) | 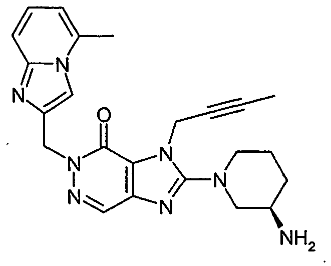 | (14) | 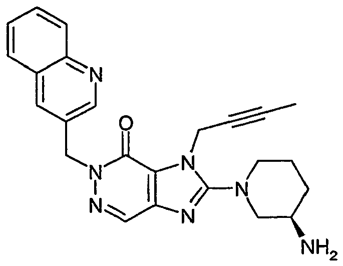 |
| | Massenspektrum: (M⁺H)⁺ = 431 | | Massenspektrum: (M+H)⁺ = 428 |

## Patentansprüche

1. Verbindungen der allgemeinen Formel 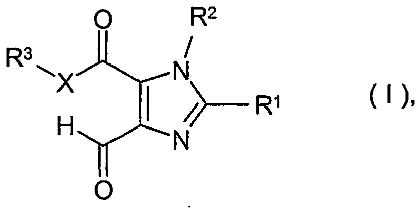 in der
R¹ ein Fluor-, Chlor-, Brom- oder lodatom,
R² eine C₃₋₈-Alkylgruppe,
eine durch eine Gruppe Rₐ substituierte C₁₋₃-Alkylgruppe, wobei
Rₐ eine gegebenenfalls durch eine oder zwei C₁₋₃-Alkylgruppen substituierte C₃₋₇-Cycloalkylgruppe,
eine gegebenenfalls durch eine oder zwei C₁₋₃-Alkylgruppen substituierte C₃₋₈-Cycloalkenylgruppe,
eine Arylgruppe oder
eine Heteroarylgruppe,
eine C₃₋₈-lkenylgruppe,
eine durch ein Fluor-, Chlor- oder Bromatom oder durch eine Trifluormethylgruppe substituierte C₃₋₆-Alkenylgruppe,
eine C₃₋₈-Alkinylgruppe,
eine Arylgruppe oder
eine Aryl-C₂₋₄-alkenylgruppe,
X ein Sauerstoff- oder Schwefelatom oder
ein Stickstoffatom, das durch R_{b} substituiert ist, wobei
R_{b} ein Wasserstoffatom,
eine Hydroxy-, Aryloxy-, Arylmethyloxy-, Heteroaryloxy-, Heteroarylmethyloxy-oder C₁₋₁₀-Alkyloxygruppe, wobei die Wasserstoffatome der Alkyloxygruppe ganz oder teilweise durch Fluoratome ersetzt sein können,
eine C₁₋₁₀-Alkylcarbonyl-, Arylcarbonyl-, Heteroarylcarbonyl-, C₁₋₁₀-Alkyloxycarbonyl-, C₁₋₁₀-Alkylaminocarbonyl-, Di-(C₁₋₁₀-alkyl)-aminocarbonyl-, C₁₋₁₀-Alkylsulfonyl-, Arylsulfonyl-, Heteroarylsulfonyl-, C₁₋₁₀-Alkylsulfinyl-, Arylsulfinyl-oder Heteroarylsulfinylgruppe, wobei die Wasserstoffatome der vorstehend genannten C₁₋₁₀-Alkylreste ganz oder teilweise durch Fluoratome ersetzt sein können,
eine C₁₋₁₀-Alkyl-, C₂₋₁₀-Alkenyl-, C₂₋₁₀-Alkinyl-, C₃₋₇-Cycloalkyl-oder C₅₋₈-Cyclo-alkenylgruppe, wobei die Wasserstoffatome in den vorstehend genannten Gruppen jeweils ganz oder teilweise durch Fluoratome ersetzt sein können und wobei in den vorstehend genannten Gruppen jeweils 1 bis 4 Methylengruppen durch ein Sauerstoff- oder Schwefelatom, durch eine -NH- oder-N(C₁₋₃-Alkyl)- Gruppe oder durch eine Carbonyl-, Sulfinyl- oder Sulfonylgruppe ersetzt sein können,
eine Arylgruppe oder
eine Heteroarylgruppe bedeutet,
oder
R_{b} und R³ miteinander verknüpft und am Stickstoffatom zu einem Ring geschlossen sind, wobei R_{b} und R³ zusammen
eine C₂₋₇-Alkylengruppe, wobei eine oder zwei Methylengruppen jeweils durch ein oder zwei Fluoratome substituiert oder durch ein Sauerstoff-oder Schwefelatom, durch eine -NH- oder -N(C₁₋₃-Alkyl)-Gruppe oder durch eine Carbonyl-, Sulfinyl- oder Sulfonylgruppe ersetzt sein können, oder eine C₄₋₇-Alkenylengruppe, wobei eine oder zwei Methylengruppen jeweils durch ein oder zwei Fluoratome substituiert oder durch ein Sauerstoff- oder Schwefelatom, durch eine -NH- oder -N(C₁₋₃-Alkyl)-Gruppe oder durch eine Carbonyl-, Sulfinyl- oder Sulfonylgruppe ersetzt sein können und wobei die Doppelbindung Teil einer an den Ring anellierten Aryl- oder Heteroarylgruppe sein kann, bedeuten,
und
R³ ein Wasserstoffatom,
eine C₁₋₂₀-Alkylgruppe, in der die Wasserstoffatome ganz oder teilweise durch Fluoratome ersetzt sein können und in der 1 bis 6.Methylengruppen jeweils durch ein Sauerstoff- oder Schwefelatom, durch eine -NH- oder -N(C₁₋₃-Alkyl)- Gruppe oder durch eine Carbonyl-, Sulfinyl- oder Sulfonylgruppe ersetzt sein können,
eine durch eine Gruppe R_{c} substituierte C₁₋₁₂-Alkylgruppe, in der die Wasserstoffatome ganz oder teilweise durch Fluoratome ersetzt sein können und in der 1 bis 4 Methylengruppen jeweils durch ein Sauerstoff- oder Schwefelatom, durch eine -NH-oder -N(C₁₋₃-Alkyl)- Gruppe oder durch eine Carbonyl-, Sulfinyl- oder Sulfonylgruppe ersetzt sein können, wobei
R_{c} eine gegebenenfalls durch eine oder zwei C₁₋₃-Alkylgruppen substituierte C₃₋₁₈-Cycloalkylgruppe, in der die Wasserstoffatome ganz oder teilweise durch Fluoratome ersetzt sein können und in der ein bis zwei Methylengruppen jeweils durch ein Sauerstoff- oder Schwefelatom, durch eine -NH- oder -N(C₁-₃-Alkyl)- Gruppe oder durch eine Carbonyl-, Sulfinyl- oder Sulfonylgruppe ersetzt sein können,
eine gegebenenfalls durch eine oder zwei C₁₋₃-Alkylgruppen substituierte C₅₋₁₈-Cycloalkenylgruppe, in der
die Wasserstoffatome ganz oder teilweise durch Fluoratome ersetzt sein können,
ein bis zwei Methylengruppen jeweils durch ein Sauerstoff- oder Schwefelatom, durch eine -NH- oder -N(C₁₋₃-Alkyl)- Gruppe oder durch eine Carbonyl-, Sulfinyl- oder Sulfonylgruppe ersetzt sein können und die Doppelbindung Bestandteil einer an den Ring anellierten Aryl- oder Heteroarylgruppe sein kann,
eine Arylgruppe oder
eine Heteroarylgruppe bedeutet,
eine C₃₋₁₈-Cycloalkylgruppe, in der die Wasserstoffatome ganz oder teilweise durch Fluoratome ersetzt sein können und in der 1 bis 6 Methylengruppen jeweils durch ein Sauerstoff- oder Schwefelatom, durch eine -NH- oder -N(C₁₋₃-Alkyl)- Gruppe oder durch eine Carbonyl-, Sulfinyl- oder Sulfonylgruppe ersetzt sein können,
eine C₃₋₂₀-Alkenylgruppe, in der die Wasserstoffatome ganz oder teilweise durch Fluoratome ersetzt sein können und in der 1 bis 6 Methylengruppen jeweils durch ein Sauerstoff- oder Schwefelatom, durch eine -NH- oder -N(C₁₋₃-Alkyl)- Gruppe oder durch eine Carbonyl-, Sulfinyl- oder Sulfonylgruppe ersetzt sein können,
eine C₅₋₂₀-Cycloalkenylgruppe, in der die Wasserstoffatome ganz oder teilweise durch Fluoratome ersetzt sein können und in der 1 bis 4 Methylengruppen jeweils durch ein Sauerstoff- oder Schwefelatom, durch eine -NH- oder -N(C₁₋₃-Alkyl)-Gruppe oder durch eine Carbonyl-, Sulfinyl- oder Sulfonylgruppe ersetzt sein können, und in der die Doppelbindung Bestandteil einer an den Ring anellierten Aryl- oder Heteroarylgruppe sein kann,
eine C₃₋₂₀-Alkinylgruppe, in der die Wasserstoffatome ganz oder teilweise durch Fluoratome ersetzt sein können und in der 1 bis 6 Methylengruppen jeweils durch ein Sauerstoff- oder Schwefelatom, durch eine -NH- oder -N(C₁₋₃-Alkyl)- Gruppe oder durch eine Carbonyl-, Sulfinyl- oder Sulfonylgruppe ersetzt sein können,
eine Arylgruppe,
eine Heteroarylgruppe,
eine Aryl-C₂₋₆-alkenylgruppe
oder, sofern X kein Stickstoffatom, das durch eine Hydroxy-, Aryloxy-, Arylmethyloxy-, Heteroaryloxy-, Heteroarylmethyloxy- oder C₁₋₁₀-Alkyloxygruppe substituiert ist, darstellt, auch eine Aminogruppe, die durch eine oder zwei C₁₋₃-Alkylgruppen substituiert sein kann,
oder, ebenfalls sofern X kein Stickstoffatom, das durch eine Hydroxy-, Aryloxy-, Arylmethyloxy-, Heteroaryloxy-, Heteroarylmethyloxy- oder C₁₋₁₀-Alkyloxygruppe substituiert ist, darstellt, auch eine 3- bis 7-gliedrigen Cycloalkyleniminogruppe, wobei ein bis zwei Methylengruppen der Cycloalkyleniminogruppe jeweils durch ein Sauerstoffatom oder eine Carbonyl-, Sulfinyl- oder Sulfonylgruppe ersetzt sein können, bedeutet oder
R³ und X zusammen ein Fluor- oder Chloratom bedeuten,
wobei unter den bei der Definition der vorstehend genannten Reste erwähnten Arylgruppen Phenyl- oder Naphthylgruppen zu verstehen sind, welche unabhängig voneinander durch Fluor- und Chloratome ein- bis fünffach substituiert und durch R_{d} mono-, di- oder trisubstituiert sein können, wobei die Substituenten gleich oder verschieden sein können und R_{d} ein Brom- oder lodatom, eine Trifluormethyl-, Cyan-, Nitro-, Amino-, Aminocarbonyl-, Aminosulfonyl-, Methylsulfonyl-, Acetylamino-, Methylsulfonylamino-, C₁₋₄-Alkyl-, C₁₋₃-Alkyl-carbonyl-, Cyclopropyl-, Ethenyl-, Ethinyl-, Hydroxy-, C₁₋₄-Alkyloxy-, C₁-₄-Alkoxy-carbonyl-, Methylsulfinyl-, Phenylsulfinyl-, Methylsulfonyl-, Phenylsulfonyl-, Difluormethoxy- oder Trifluormethoxygruppe darstellt,
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Chinolinyl- oder Isochinolinylgruppe
oder eine Pyrrolyl-, Furanyl-, Thienyl- oder Pyridylgruppe in der eine oder zwei Methingruppen durch Stickstoffatome ersetzt sind,
oder eine Indolyl-, Benzofuranyl-, Benzothiophenyl-, Chinolinyl- oder Isochinolinylgruppe in der eine bis drei Methingruppen durch Stickstoffatome ersetzt sind, zu verstehen ist,
und die vorstehend erwähnten Heteroarylgruppen durch Fluor- und Chloratome ein- bis fünffach substituiert und R_{d} mono-, di- oder trisubstituiert sein können, wobei die Substituenten gleich oder verschieden sein können und R_{d} wie vorstehend erwähnt definiert ist,
unter den bei der Definition der vorstehend erwähnten Cycloalkylgruppen sowohl mono- als auch polycyclische Ringsysteme, die entweder verbrückt, spiro-verknüpft oder anelliert aufgebaut sind, zu verstehen sind,
unter den bei der Definition der vorstehend erwähnten Cycloalkenylgruppen sowohl mono- als auch polycyclische Ringsysteme, die entweder verbrückt oder anelliert aufgebaut sind, und die mindestens eine C=C-Doppelbindung tragen, zu verstehen sind,
wobei, soweit nichts anderes erwähnt wurde, die vorstehend erwähnten Alkyl-, Alkenyl- und Alkinylgruppen geradkettig oder verzweigt sein können,
wobei die Verbindung, in der R¹ ein Bromatom, R² eine 2-Butinylgruppe, X ein Sauerstoffatom und R³ eine Methylgruppe bedeuten, ausgeschlossen ist,
deren Tautomere, deren Enantiomere, deren Diastereomere, deren Gemische und deren Salze.

2. Verbindungen der allgemeinen Formel I gemäß Anspruch 1, in denen
R² und R³ wie in Anspruch 1 erwähnt definiert sind,
R¹ ein Chlor- oder Bromatom und
X ein Sauerstoffatom oder eine -NH- oder -N(C₁₋₃-Alkyl)- Gruppe bedeuten,
wobei die Verbindung, in der R¹ ein Bromatom, R² eine 2-Butinylgruppe, X ein Sauerstoffatom und R³ eine Methylgruppe bedeuten, ausgeschlossen ist,
deren Tautomere, deren Enantiomere, deren Diastereomere, deren Gemische und deren Salze.

3. Verbindungen der allgemeinen Formel I gemäß Anspruch 2, in denen
R¹ ein Chlor- oder Bromatom,
R² eine durch eine Gruppe Rₐ substituierte C₁₋₃-Alkylgruppe, wobei Rₐ wie in Anspruch 1 erwähnt definiert ist,
eine C₃₋₈-Alkenylgruppe,
eine durch ein Fluor-, Chlor- oder Bromatom oder durch eine Trifluormethylgruppe substituierte C₃₋₆-Alkenylgruppe oder
eine C₃₋₈-Alkinylgruppe,
X ein Sauerstoffatom und
R³ ein Wasserstoffatom,
eine C₁₋₂₀-Alkylgruppe, in der die Wasserstoffatome ganz oder teilweise durch Fluoratome ersetzt sein können und in der 1 bis 6 Methylengruppen jeweils durch ein Sauerstoff- oder Schwefelatom, durch eine -NH- oder -N(C₁₋₃-Alkyl)- Gruppe oder durch eine Carbonyl-, Sulfinyl- oder Sulfonylgruppe ersetzt sein können,
eine durch eine Gruppe R_{c} substituierte C₁₋₁₂-Alkylgruppe, in der die Wasserstoffatome ganz oder teilweise durch Fluoratome ersetzt sein können und in der 1 bis 4 Methylengruppen jeweils durch ein Sauerstoff- oder Schwefelatom, durch eine -NH-oder -N(C₁₋₃-Alkyl)- Gruppe oder durch eine Carbonyl-, Sulfinyl- oder Sulfonylgruppe ersetzt sein können, und wobei R_{c} wie oben erwähnt definiert ist,
eine C₃₋₈-Cycloalkylgruppe, in der die Wasserstoffatome ganz oder teilweise durch Fluoratome ersetzt sein können und in der 1 bis 4 Methylengruppen jeweils durch ein Sauerstoff- oder Schwefelatom, durch eine -NH- oder -N(C₁₋₃-Alkyl)- Gruppe oder durch eine Carbonyl-, Sulfinyl- oder Sulfonylgruppe ersetzt sein können,
eine C₃₋₂₀-Alkenylgruppe, in der die Wasserstoffatome ganz oder teilweise durch Fluoratome ersetzt sein können und in der 1 bis 6 Methylengruppen jeweils durch ein Sauerstoff- oder Schwefelatom, durch eine -NH- oder -N(C₁₋₃-Alkyl)- Gruppe oder durch eine Carbonyl-, Sulfinyl- oder Sulfonylgruppe ersetzt sein können,
eine C₃₋₂₀-Alkinylgruppe, in der die Wasserstoffatome ganz oder teilweise durch Fluoratome ersetzt sein können und in der 1 bis 6 Methylengruppen jeweils durch ein Sauerstoff oder Schwefelatom, durch eine -NH- oder -N(C₁₋₃-Alkyl)- Gruppe oder durch eine Carbonyl-, Sulfinyl- oder Sulfonylgruppe ersetzt sein können, oder
eine Aryl-C₂₋₆-alkenylgruppe bedeuten,
wobei die Verbindung, in der R¹ ein Bromatom, R² eine 2-Butinylgruppe, X ein Sauerstoffatom und R³ eine Methylgruppe bedeuten, ausgeschlossen ist,
deren Tautomere, deren Enantiomere, deren Diastereomere, deren Gemische und deren Salze.

4. Verbindungen der allgemeinen Formel I gemäß Anspruch 3, in denen
R¹ ein Chlor- oder Bromatom,
R² eine Phenylmethylgruppe, die am Phenylring durch ein Fluor-, Chlor-, Brom- oder lodatom oder durch eine Cyano- oder Methoxygruppe substituiert sein kann,
eine C₃₋₈-Alkenylgruppe,
eine C₃₋₈-Cycloalkenylmethylgruppe,
eine durch ein Fluor-, Chlor- oder Bromatom oder durch eine Trifluormethylgruppe substituierte C₃₋₆-Alkenylgruppe oder
eine C₃₋₈-Alkinylgruppe,
X ein Sauerstoffatom und
R³ ein Wasserstoffatom,
eine C₁-₂₀-Alkylgruppe, in der die Wasserstoffatome ganz oder teilweise durch Fluoratome ersetzt sein können und in der 1 bis 6 Methylengruppen jeweils durch ein Sauerstoff- oder Schwefelatom oder durch eine -NH- oder -N(C₁₋₃-Alkyl)- Gruppe ersetzt sein können,
eine durch eine Gruppe R_{c} substituierte C₁₋₁₂-Alkylgruppe, in der die Wasserstoffatome ganz oder teilweise durch Fluoratome ersetzt sein können und in der 1 bis 4 Methylengruppen jeweils durch ein Sauerstoff- oder Schwefelatom oder durch eine -NH- oder -N(C₁₋₃-Alkyl)- Gruppe ersetzt sein können, und wobei R_{c} wie oben erwähnt definiert ist,
eine C₃₋₈-Cycloalkylgruppe, in der die Wasserstoffatome ganz oder teilweise durch Fluoratome ersetzt sein können und in der 1 bis 4 Methylengruppen jeweils durch ein Sauerstoff- oder Schwefelatom oder durch eine -NH- oder -N(C₁₋₃-Alkyl)- Gruppe ersetzt sein können,
eine C₃₋₂₀-Alkenylgruppe oder
eine C₃₋₂₀-Alkinylgruppe bedeuten,
wobei die Verbindung, in der R¹ ein Bromatom, R² eine 2-Butinylgruppe, X ein Sauerstoffatom und R³ eine Methylgruppe bedeuten, ausgeschlossen ist,
deren Tautomere, deren Enantiomere, deren Diastereomere, deren Gemische und deren Salze.

5. Verbindungen der allgemeinen Formel I gemäß Anspruch 4, in denen
R¹ ein Bromatom,
R² eine 2-Buten-1-yl- oder 3-Methyl-2-buten-1-yl-Gruppe,
eine 2-Butin-1-yl-Gruppe oder
eine 2-Chlorphenylmethyl- oder 2-Bromphenylmethyl-Gruppe,
X ein Sauerstoffatom,
und
R³ eine C₁₋₁₀-Alkylgruppe oder C₃₋₈-Cycloalkylgruppe bedeuten,
wobei die Verbindung, in der R¹ ein Bromatom, R² eine 2-Butinylgruppe, X ein Sauerstoffatom und R³ eine Methylgruppe bedeuten, ausgeschlossen ist,
deren Tautomere, deren Enantiomere, deren Diastereomere, deren Gemische und deren Salze.

6. Folgende Verbindungen der allgemeinen Formel I gemäß Anspruch 5:
(a) 2-Brom-5-formyl-3-(3-methyl-2-buten-1-yl)-3H-imidazol-4-carbonsäuremethylester,
(b) 2-Brom=5-formyl-3-(2-butin-1-yl)-3H-imidazol-4-carbonsäureethylester
sowie deren Salze.

7. Verfahren zur Herstellung der Verbindungen I gemäß Anspruch 1, **dadurch gekennzeichnet, daß**
a) eine Verbindung der allgerneinen Formel 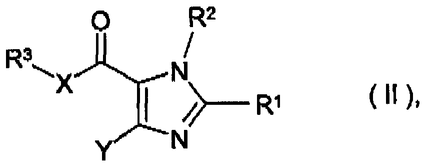 in der
R¹ bis R³ und X wie in den Ansprüchen 1 bis 6 erwähnt definiert sind und Y eine Carbonsäure-, Carbonsäureamid-, Nitril-, Carbonsäureester-, Carbonsäurethioester-, Carbonsäureanhydrid- oder Carbonsäurechlorid-Gruppe, die jeweils über das Carboxylkohlenstoffatom an den Imidazolring gebunden sind, bedeutet, reduziert wird,
b) eine Verbindung der allgemeinen Formel 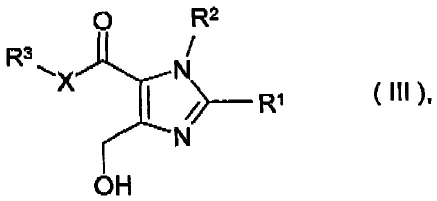 in der R¹, R², R³ und X wie in Anspruch 1 bis 6 erwähnt definiert sind, oxidiert wird.

8. Verfahren zur Herstellung von Imidazo[4,5-d]pyridazin-4-onen der allgemeinen Formel 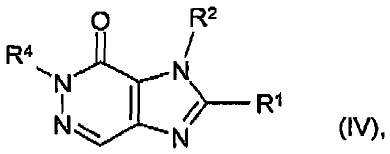 in der R¹ und R² wie in den Ansprüchen 1 bis 6 erwähnt definiert sind,
**dadurch gekennzeichnet, dass** eine Verbindung der allgemeinen Formel I, in der R¹ bis R³ und X wie in den Ansprüchen 1 bis 6 erwähnt definiert sind, mit einem durch R⁴ substituierten Hydrazin oder einem entsprechenden geschützten Hydrazinderivat, wobei,
R⁴ ein Wasserstoffatom,
eine C₃₋₈-Alkylgruppe,
eine durch eine Gruppe Rₐ substituierte C₁₋₃-Alkygruppe, wobei Rₐ wie in Anspruch 1 definiert ist
eine C₃₋₈-Alkenyl- oder C₃₋₈-Alkinylgruppe,
eine Arylcarbonylmethyl- oder Heteroarylcarbonylmethylgruppe,
eine Arylprop-2-enyl- oder Heteroarylprop-2-enylgruppe oder
eine Arylgruppe oder Heteroarylgruppe bedeutet,
umgesetzt wird,
und gegebenenfalls die Reste R¹ und/oder R² der so erhaltenen Verbindung der allgemeinen Formel IV verändert oder weitere Substituenten in das Grundgerüst eingebracht werden
und, falls gewünscht, die Verbindung der Formel IV in ihre Salze mit organischen oder anorganischen Säuren oder Basen überführt wird.

9. Verfahren zur Herstellung von Imidazo[4,5-d]pyridazin-4-onen der allgemeinen Formel 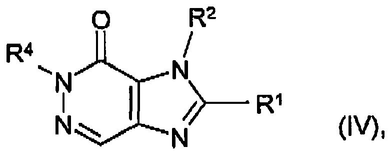 in der R¹ und R² wie in den Ansprüchen 1 bis 6 erwähnt definiert sind,
**dadurch gekennzeichnet, dass**
a) eine Verbindung der allgemeinen Formel 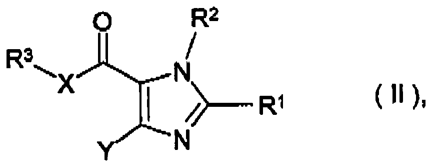 in der
R¹ bis R³ und X wie in den Ansprüchen 1 bis 6 erwähnt definiert sind und Y eine Carbonsäure-, Carbonsäureamid-, Nitril-, Carbonsäureester-, Carbonsäurethioester-, Carbonsäureanhydrid- oder Carbonsäurechlorid-Gruppe, die jeweils über das Carboxylkohlenstoffatom an den Imidazolring gebunden sind, bedeutet, reduziert wird,
oder
b) eine Verbindung der allgemeinen Formel 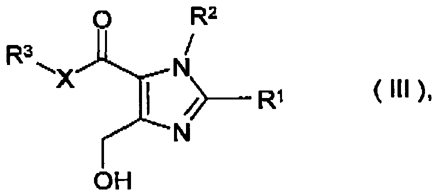
in der R¹, R², R³ und X wie in Anspruch 1 bis 6 erwähnt definiert sind, oxidiert wird, und c) eine gemäß Schritt a) oder b) erhaltenene Verbindung der allgemeinen Formel 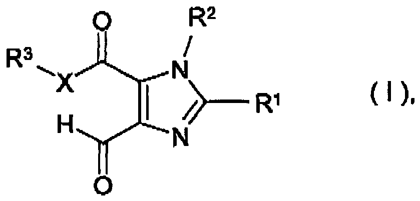 in der R¹ bis R³ und X wie in den Ansprüchen 1 bis 6 erwähnt definiert sind, mit einem durch R⁴ substituierten Hydrazin oder einem entsprechenden geschützten Hydrazinderivat umgesetzt wird, wobei
R⁴ ein Wasserstoffatom,
eine C₃₋₈-Alkygruppe,
eine durch eine Gruppe Rₐ substituierte C₁₋₃-Alkylgruppe, wobei Rₐ wie in Anspruch 1 definiert ist,
eine C₃₋₈-Alkenyl- oder C₃₋₈-Alkinylgruppe,
eine Arylcarbonylmethyl- oder Heteroarylcarbonylmethylgruppe,
eine Arylprop-2-enyl- oder Heteroarylprop-2-enylgrupppe oder
eine Arylgruppe oder Heteroarylgruppe bedeutet,
und gegebenenfalls die Reste R¹ und/oder R² der so erhaltenen Verbindung der allgemeinen Formel IV verändert oder weitere Substituenten in das Grundgerüst eingebracht werden
und, falls gewünscht, die Verbindung der Formel IV in ihre Salze mit organischen oder anorganischen Säuren oder Basen überführt wird.

## Claims

1. Compounds of general formula 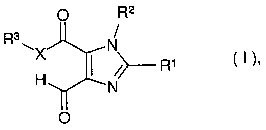 wherein
R¹ denotes a fluorine, chlorine, bromine or iodine atom,
R² denotes a C₃₋₈-alkyl group,
a C₁₋₃-alkyl group substituted by a group Rₐ, while
Rₐ denotes a C₃₋₇-cycloalkyl group optionally substituted by one or two C₁₋₃-alkyl groups,
a C₃₋₈-cycloalkenyl group optionally substituted by one or two C₁₋₃-alkyl groups,
an aryl group or
a heteroaryl group,
a C₃₋₈-alkenyl group,
a C₃₋₆-alkenyl group substituted by a fluorine, chlorine or bromine atom or by a trifluoromethyl group,
a C₃₋₈-alkynyl group,
an aryl group or
an aryl-C₂₋₄-alkenyl group,
X denotes an oxygen or sulphur atom or
a nitrogen atom which is substituted by R_{b}, while
R_{b} denotes a hydrogen atom,
a hydroxy, aryloxy, arylmethyloxy, heteroaryloxy, heteroarylmethyloxy or C₁₋₁₀-alkyloxy group, while the hydrogen atoms of the alkyloxy group may be wholly or partly replaced by fluorine atoms,
a C₁₋₁₀-alkylcarbonyl, arylcarbonyl, heteroarylcarbonyl, C₁₋₁₀-alkyloxycarbonyl, C₁₋₁₀-alkylaminocarbonyl, di-(C₁₋₁₀-alkyl)-aminocarbonyl, C₁₋₁₀-alkylsulphonyl, arylsulphonyl, heteroarylsulphonyl, C₁₋₁₀-alkylsulphinyl, arylsulphinyl or heteroarylsulphinyl group, while the hydrogen atoms of the above-mentioned C₁₋₁₀-alkyl groups may be wholly or partly replaced by fluorine atoms,
a C₁₋₁₀-alkyl, C₂₋₁₀-alkenyl, C₂₋₁₀-alkynyl, C₃₋₇-cycloalkyl or C₅₋₈-cycloalkenyl group, while the hydrogen atoms in the above-mentioned groups may each be wholly or partly replaced by fluorine atoms and in the above-mentioned groups 1 to 4 methylene groups may each be replaced by an oxygen or sulphur atom, by an -NH- or -N(C₁₋₃-alkyl)- group or by a carbonyl, sulphinyl or sulphonyl group,
an aryl group or
a heteroaryl group,
or
R_{b} and R³ are linked together and are closed up into a ring at the nitrogen atom, while R_{b} and R³ together denote
a C₂₋₇-alkylene group, while one or two methylene groups may each be substituted by one or two fluorine atoms or replaced by an oxygen or sulphur atom, by an -NH- or -N(C₁₋₃-alkyl)- group or by a carbonyl, sulphinyl or sulphonyl group,
or a C₄₋₇-alkenylene group, while one or two methylene groups may each be substituted by one or two fluorine atoms or replaced by an oxygen or sulphur atom, by an -NH- or -N(C₁₋₃-alkyl)- group or by a carbonyl, sulphinyl or sulphonyl group and the double bond may be part of an aryl or heteroaryl group anellated to the ring,
and
R³ denotes a hydrogen atom,
a C₁₋₂₀-alkyl group wherein the hydrogen atoms may be wholly or partly replaced by fluorine atoms and wherein 1 to 6 methylene groups may each be replaced by an oxygen or sulphur atom, by an -NH- or -N(C₁₋₃-alkyl)- group or by a carbonyl, sulphinyl or sulphonyl group,
a C₁₋₁₂-alkyl group substituted by a group R_{c} wherein the hydrogen atoms may be wholly or partly replaced by fluorine atoms and wherein 1 to 4 methylene groups may each be replaced by an oxygen or sulphur atom, by an -NH- or -N(C₁₋₃-alkyl)-group or by a carbonyl, sulphinyl or sulphonyl group, while
R_{c} denotes a C₃₋₁₈-cycloalkyl group optionally substituted by one or two C₁₋₃-alkyl groups wherein the hydrogen atoms may be wholly or partly replaced by fluorine atoms and wherein one to two methylene groups may each be replaced by an oxygen or sulphur atom, by an -NH- or -N(C₁₋₃-alkyl)-group or by a carbonyl, sulphinyl or sulphonyl group,
a C₅₋₁₈-cycloalkenyl group optionally substituted by one or two C₁₋₃-alkyl groups, wherein
the hydrogen atoms may be wholly or partly replaced by fluorine atoms,
one to two methylene groups may each be replaced by an oxygen or sulphur atom, by an -NH- or -N(C₁₋₃-alkyl)- group or by a carbonyl, sulphinyl or sulphonyl group and
the double bond may be part of an aryl or heteroaryl group anellated to the ring,
an aryl group or
a heteroaryl group,
a C₃₋₁₈-cycloalkyl group wherein the hydrogen atoms may be wholly or partly replaced by fluorine atoms and wherein 1 to 6 methylene groups may each be replaced by an oxygen or sulphur atom, by an -NH- or-N(C₁₋₃-alkyl)- group or by a carbonyl, sulphinyl or sulphonyl group,
a C₃₋₂₀-alkenyl group wherein the hydrogen atoms may be wholly or partly replaced by fluorine atoms and wherein 1 to 6 methylene groups may each be replaced by an oxygen or sulphur atom, by an -NH- or -N(C₁₋₃-alkyl)- group or by a carbonyl, sulphinyl or sulphonyl group,
a C₅₋₂₀-cycloalkenyl group wherein the hydrogen atoms may be wholly or partly replaced by fluorine atoms and wherein 1 to 4 methylene groups may each be replaced by an oxygen or sulphur atom, by an -NH- or-N(C₁₋₃-alkyl)- group or by a carbonyl, sulphinyl or sulphonyl group,
and wherein the double bond may be part of an aryl or heteroaryl group anellated to the ring,
a C₃₋₂₀-alkynyl group wherein the hydrogen atoms may be wholly or partly replaced by fluorine atoms and wherein 1 to 6 methylene groups may each be replaced by an oxygen or sulphur atom, by an -NH- or -N(C₁₋₃-alkyl)- group or by a carbonyl, sulphinyl or sulphonyl group,
an aryl group,
a heteroaryl group,
an aryl-C₂₋₆-alkenyl group
or, if X does not represent a nitrogen atom which is substituted by a hydroxy, aryloxy, arylmethyloxy, heteroaryloxy, heteroarylmethyloxy or C₁₋₁₀-alkyloxy group, may also denote an amino group which may be substituted by one or two C₁₋₃-alkyl groups,
or, again if X does not represent a nitrogen atom which is substituted by a hydroxy, aryloxy, arylmethyloxy, heteroaryloxy, heteroarylmethyloxy or C₁₋₁₀-alkyloxy group, may also denote a 3- to 7-membered cycloalkyleneimino group, while one to two methylene groups of the cycloalkyleneimino group may each be replaced by an oxygen atom or a carbonyl, sulphinyl or sulphonyl group, or
R³ and X together represent a fluorine or chlorine atom,
while by the aryl groups mentioned in the definition of the above groups are meant phenyl or naphthyl groups which may be mono- to pentasubstituted independently of one another by fluorine and chlorine atoms and may be mono-, di- or trisubstituted by R_{d}, while the substituents may be identical or different and R_{d} denotes a bromine or iodine atom, a trifluoromethyl, cyano, nitro, amino, aminocarbonyl, aminosulphonyl, methylsulphonyl, acetylamino, methylsulphonylamino, C₁₋₄-alkyl, C₁₋₃-alkyl-carbonyl, cyclopropyl, ethenyl, ethynyl, hydroxy, C₁₋₄-alkyloxy, C₁₋₄-alkoxycarbonyl, methylsulphinyl, phenylsulphinyl, methylsulphonyl, phenylsulphonyl, difluoromethoxy or trifluoromethoxy group,
by the heteroaryl groups mentioned in the definition of the above-mentioned groups are meant a pyrrolyl, furanyl, thienyl, pyridyl, indolyl, benzofuranyl, benzothiophenyl, quinolinyl or isoquinolinyl group
or a pyrrolyl, furanyl, thienyl or pyridyl group wherein one or two methyne groups are replaced by nitrogen atoms,
or an indolyl, benzofuranyl, benzothiophenyl, quinolinyl or isoquinolinyl group wherein one to three methyne groups are replaced by nitrogen atoms,
and the above-mentioned heteroaryl groups may be mono- to pentasubstituted by fluorine and chlorine atoms and R_{d} may be mono-, di- or trisubstituted, while the substituents may be identical or different and R_{d} is as hereinbefore defined,
by the cycloalkyl groups mentioned in the above definitions are meant both mono-and polycyclic ring systems, which are either bridged, spiro-bridged or anellated in construction,
by the cycloalkenyl groups mentioned in the above definitions are meant both mono-and polycyclic ring systems, which are either bridged or anellated in construction, and have at least one C=C double bond,
while, unless otherwise stated, the above-mentioned alkyl, alkenyl and alkynyl groups may be straight-chain or branched,
with the exclusion of the compound wherein R¹ denotes a bromine atom, R² denotes a 2-butynyl group, X denotes an oxygen atom and R³ denotes a methyl group,
the tautomers, the enantiomers, the diastereomers, the mixtures thereof and the salts thereof.

2. Compounds of general formula I according to claim 1, wherein
R² and R³ are defined as in claim 1,
R¹ denotes a chlorine or bromine atom and
X denotes an oxygen atom or an -NH- or -N(C₁₋₃-alkyl)- group,
with the exclusion of the compound wherein R¹ denotes a bromine atom, R² denotes a 2-butynyl group, X denotes an oxygen atom and R³ denotes a methyl group,
the tautomers, the enantiomers, the diastereomers, the mixtures thereof and the salts thereof.

3. Compounds of general formula I according to claim 2, wherein
R¹ denotes a chlorine or bromine atom,
R² denotes a C₁₋₃-alkyl group substituted by a group Rₐ, where Rₐ is as hereinbefore defined,
a C₃₋₈-alkenyl group,
a C₃₋₆-alkenyl group substituted by a fluorine, chlorine or bromine atom or by a trifluoromethyl group or
a C₃₋₈-alkynyl group,
X denotes an oxygen atom and
R³ denotes a hydrogen atom,
a C₁₋₂₀-alkyl group wherein the hydrogen atoms may be wholly or partly replaced by fluorine atoms and wherein 1 to 6 methylene groups may each be replaced by an oxygen or sulphur atom, by an -NH- or -N(C₁₋₃-alkyl)- group or by a carbonyl, sulphinyl or sulphonyl group,
a C₁₋₁₂-alkyl group substituted by a group R_{c} wherein the hydrogen atoms may be wholly or partly replaced by fluorine atoms and wherein 1 to 4 methylene groups may each be replaced by an oxygen or sulphur atom, by an -NH- or -N(C₁₋₃-alkyl)-group or by a carbonyl, sulphinyl or sulphonyl group, where R_{c} is as hereinbefore defined,
a C₃₋₈-cycloalkyl group wherein the hydrogen atoms may be wholly or partly replaced by fluorine atoms and wherein 1 to 4 methylene groups may each be replaced by an oxygen or sulphur atom, by an -NH- or -N(C₁₋₃-alkyl)- group or by a carbonyl, sulphinyl or sulphonyl group,
a C₃₋₂₀-alkenyl group wherein the hydrogen atoms may be wholly or partly replaced by fluorine atoms and wherein 1 to 6 methylene groups may each be replaced by an oxygen or sulphur atom, by an -NH- or -N(C₁₋₃-alkyl)- group or by a carbonyl, sulphinyl or sulphonyl group,
a C₃₋₂₀-alkynyl group wherein the hydrogen atoms may be wholly or partly replaced by fluorine atoms and wherein 1 to 6 methylene groups may each be replaced by an oxygen or sulphur atom, by an -NH- or -N(C₁₋₃-alkyl)- group or by a carbonyl, sulphinyl or sulphonyl group, or
an aryl-C₂₋₆-alkenyl group,
with the exclusion of the compound wherein R¹ denotes a bromine atom, R² denotes a 2-butynyl group, X denotes an oxygen atom and R³ denotes a methyl group,
the tautomers, the enantiomers, the diastereomers, the mixtures thereof and the salts thereof.

4. Compounds of general formula I according to claim 3, wherein
R¹ denotes a chlorine or bromine atom,
R² denotes a phenylmethyl group which may be substituted at the phenyl ring by a fluorine, chlorine, bromine or iodine atom or by a cyano or methoxy group,
a C₃₋₈-alkenyl group,
a C₃₋₈-cycloalkenylmethyl group,
a C₃₋₈-alkenyl group substituted by a fluorine, chlorine or bromine atom or by a trifluoromethyl group or
a C₃₋₈-alkynyl group,
X denotes an oxygen atom and
R³ denotes a hydrogen atom,
a C₁₋₂₀-alkyl group wherein the hydrogen atoms may be wholly or partly replaced by fluorine atoms and wherein 1 to 6 methylene groups may each be replaced by an oxygen or sulphur atom or by an -NH- or -N(C₁₋₃-alkyl)- group,
a C₁₋₁₂-alkyl group substituted by a group R_{c} wherein the hydrogen atoms may be wholly or partly replaced by fluorine atoms and wherein 1 to 4 methylene groups may each be replaced by an oxygen or sulphur atom or by an -NH- or -N(C₁₋₃-alkyl)-group, and where R_{c} is as hereinbefore defined,
a C₃₋₈-cycloalkyl group wherein the hydrogen atoms may be wholly or partly replaced by fluorine atoms and wherein 1 to 4 methylene groups may each be replaced by an oxygen or sulphur atom or by an -NH- or-N(C₁₋₃-alkyl)- group,
a C₃₋₂₀-alkenyl group or
a C₃₋₂₀-alkynyl group,
with the exclusion of the compound wherein R¹ denotes a bromine atom, R² denotes a 2-butynyl group, X denotes an oxygen atom and R³ denotes a methyl group,
the tautomers, the enantiomers, the diastereomers, the mixtures thereof and the salts thereof.

5. Compounds of general formula I according to claim 4, wherein
R¹ denotes a bromine atom,
R² denotes a 2-buten-1-yl or 3-methyl-2-buten-1-yl group,
a 2-butyn-1-yl group or
a 2-chlorophenylmethyl or 2-bromophenylmethyl group,
X denotes an oxygen atom,
and
R³ denotes a C₁₋₁₀-alkyl group or C₃₋₈-cycloalkyl group,
with the exclusion of the compound wherein R¹ denotes a bromine atom, R² denotes a 2-butynyl group, X denotes an oxygen atom and R³ denotes a methyl group,
the tautomers, the enantiomers, the diastereomers, the mixtures thereof and the salts thereof.

6. The following compounds of general formula I according to claim 5:
(a) methyl 2-bromo-5-formyl-3-(3-methyl-2-buten-1-yl)-3*H*-imidazole-4-carboxylate,
(b) ethyl 2-bromo-5-formyl-3-(2-butyn-1-yl)-3*H*-imidazole-4-carboxylate
and the salts thereof.

7. Process for preparing the compounds I according to claim 1, **characterised in that**
a) a compound of general formula 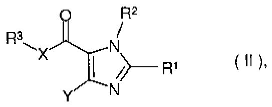 wherein
R¹ to R³ and X are defined as in claims 1 to 6 and Y denotes a carboxylic acid, carboxylic acid amide, nitrile, carboxylic acid ester, carboxylic acid thioester, carboxylic acid anhydride or carboxylic acid chloride group, each of which is bound to the imidazole ring via the carboxyl carbon atom, is reduced,
b) a compound of general formula 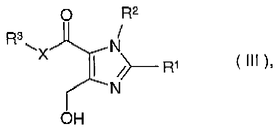
wherein R¹, R², R³ and X are defined as mentioned in claims 1 to 6, is oxidised.

8. Process for preparing imidazo[4,5-d]pyridazin-4-ones of general formula 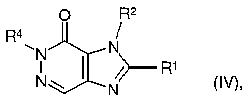 wherein R¹ and R² are defined as in claims 1 to 6,
**characterised in that** a compound of general formula I wherein R¹ to R³ and X are defined as in claims 1 to 6, is reacted with a hydrazine substituted by R⁴ or a correspondingly protected hydrazine derivative, wherein
R¹ denotes a hydrogen atom,
a C₃₋₈-alkyl group,
a C₁₋₃-alkyl group substituted by a group Rₐ, where Rₐ is defined as in claim 1,
a C₃₋₈-alkenyl or C₃₋₈-alkynyl group,
an arylcarbonylmethyl or heteroarylcarbonylmethyl group,
an arylprop-2-enyl or heteroarylprop-2-enyl group or
an aryl group or heteroaryl group,
and optionally the groups R¹ and/or R² of the compound of general formula IV thus obtained are changed or other substituents are introduced into the basic structure
and, if desired, the compound of formula IV is converted into the salts thereof with organic or inorganic acids or bases.

9. Process for preparing imidazo[4,5-d]pyridazin-4-ones of general formula 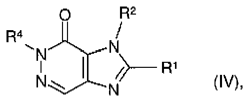 wherein R¹ and R² are defined as in claims 1 to 6,
**characterised in that**
a) a compound of general formula 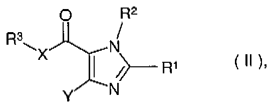 wherein
R¹ to R³ and X are defined as in claims 1 to 6 and Y denotes a carboxylic acid, carboxylic acid amide, nitrile, carboxylic acid ester, carboxylic acid thioester, carboxylic acid anhydride or carboxylic acid chloride group each of which is bound to the imidazole ring via the carboxyl carbon atom, is reduced,
or
b) a compound of general formula 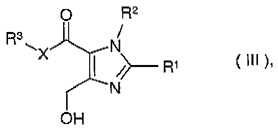
wherein R¹, R², R³ and X are defined as in claims 1 to 6, is oxidised,
and c) a compound of general formula 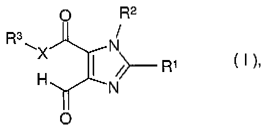 obtained according to step a) or b),
wherein R¹ to R³ and X are defined as in claims 1 to 6, is reacted with a hydrazine substituted by R⁴ or a correspondingly protected hydrazine derivative, wherein
R⁴ denotes a hydrogen atom,
a C₃₋₈-alkyl group,
a C₁₋₃-alkyl group substituted by a group Rₐ, where Rₐ is defined as in claim 1,
a C₃₋₅-alkenyl or C₃₋₈-alkynyl group,
an arylcarbonylmethyl or heteroarylcarbonylmethyl group,
an arylprop-2-enyl or heteroarylprop-2-enyl group or
an aryl group or heteroaryl group,
and optionally the groups R¹ and/or R² of the compound of general formula IV thus obtained are changed or other substituents are introduced into the basic structure
and, if desired, the compound of formula IV is converted into the salts thereof with organic or inorganic acids or bases.

## Revendications

1. Composés de formule générale : 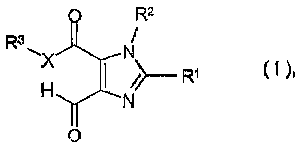 dans laquelle
R¹ représente un atome de fluor, de chlore, de brome ou d'iode,
R⁷ représente un groupe allyle en C₃ à C₈,
un groupe allyle en C₁ à C₃ substitués par un groupe Rₐ, dans lequel
Rₐ est un groupe cycloalkyle en C₃ à C₇ éventuellement substitué par un ou deux groupes alkyle en C₁ à C₃,
un groupe cycloalcényle en C₃ à C₈ substitué par un ou deux groupe alkyle en C₁ à C₃,
un groupe aryle ou
un groupe hétéroaryle,
un groupe alcényle en C₃ à C₈,
un groupe alcényle en C₃ à C₆ substitué par un atome de fluor, de chlore ou de brome ou par un groupe trifluorométhyle,
un groupe alcinyle en C₃ à C₈,
un groupe aryle ou
un groupe aryl-alcényle en C₂ à C₄,
X est un atome d'oxygène ou de souffre ou
un atome d'azote qui est substitué par R_{b},
R_{b} étant un atome d'hydrogène,
un groupe hydroxy, aryloxy, arylméthyloxy, hétéroaryloxy, hétéroarylméthoxy ou alkyloxy en C₁ à C₁₀, les atomes d'hydrogène du groupe alkyloxy pouvant être substitués totalement ou partiellement par des atomes de fluor,
un groupe alkylcarbonyle en C₁ à C₁₀, arylcarbonyle, hétéroarylcarbonyle, alkyloxy en C₁ à C₁₀-carbonyle, alkyleminocarbonyle en C₁ à C₁₀, di-(alkyle en C₁ à C₁₀)-aminocarbonyle, alkylsulfonyle en C₁ à C₁₀, arylsulfonyle, hétéroarylsulfonyle, alkylsulfinyle en C₁ à C₁₀, arylsulfinyle ou hétéroarylsulfinyle, les atomes d'hydrogène des radicaux alkyle en C₁ à C₁₀ précédemment cités pouvant être remplacées totalement ou partiellement par des atomes de fluor,
un groupe alkyle en C₁ à C₁₀, alcényle en C₂ à C₁₀, alcinyle en C₂ à C₁₀, cycloalkyle en C₃ à C₇ ou cyclo en C₅ à C₈-alcényle, les atomes d'hydrogène pouvant être respectivement substitués totalement ou partiellement dans les groupes précédemment cités par des atomes de fluor et dans les groupes précédemment cités, respectivement 1 à 4 groupes éthylène pouvant être substitués par un atome d'oxygène ou de soufre, par un groupe -NH ou N(alkyle en C₁ à C₃) ou par un groupe carbonyle, sulfinyle ou sulfonyle,
un groupe aryle ou
un groupe hétéroaryle
ou
R_{b} et R³ sont reliés l'un à l'autre et fermés en un cycle au niveau de l'atome d'azote, R_{b} et R³ conjointement désignent
un groupe alcylène en C₂ à C₇, dans lequel un ou deux groupes méthylène peuvent être substitués respectivement par un ou deux atomes de fluor ou par un atome d'oxygène ou de soufre, par un groupe -NH ou - N(alkyle en C₁ à C₃) ou par un groupe carbonyle, sulfinyle ou sulfonyle,
ou par un groupe alcénylène en C₄ à C₇, dans lequel un ou deux groupes méthylène peuvent être substitués respectivement par un ou deux atomes de fluor ou par un atome d'oxygène ou de soufre, par un groupe -NH ou -N(alkyle en C₁ à C₃) ou par un groupe carbonyle, sulfinyle ou sulfonyle et dans lequel la double liaison peut être une partie d'un groupe aryle ou hétéroaryle annelé au cycle,
et
R³ représente un atome d'hydrogène,
un groupe alkyle en C₁ à C₂₀ dans lequel les atomes d'hydrogène peuvent être substitués totalement ou partiellement par des atomes de fluor et dans lequel 1 à 6 groupes méthylène peuvent être remplacés respectivement par un atome d'oxygène ou de soufre, par un groupe -NH ou -N(alkyle en C₁ à C₃) ou par un groupe carbonyle, sulfinyle ou sulfonyle,
un groupe alkyle en C₁ à C₁₃ substitué par un groupe R_{c} dans lequel les atomes d'hydrogène peuvent être substitués totalement ou partiellement par des atomes de fluor et dans lequel 1 à 4 groupes méthylène peuvent être substitués respectivement par un atome d'oxygène ou de soufre, par un groupe -NH ou -N(alkyle en C₁ à C₃) ou par un groupe carbonyle, sulfonyle ou sulfonyle, dans lequel
R_{c} désigne un groupe cycloalkyle en C₃ à C₁₀ éventuellement substitué par un ou deux groupes alkyle en C₁ à C₃, dans lequel les atomes d'hydrogène peuvent être substitués totalement ou partiellement par des atomes de fluor et dans lequel un à deux groupes méthylène peuvent être substitués respectivement par un atome d'oxygène ou de soufre, par un groupe -NH ou - N (alkyle en C₁ à C₃) ou par un groupe carbonyle, sulfinyle ou sulfonyle,
un groupe cycloalcényle en C₅ à C₁₈ éventuellement substitué par un ou deux groupes alkyle en C₁ à C₃, dans lequel
les atomes d'hydrogène peuvent être substitués totalement ou partiellement par des atomes de fluor,
un à deux groupes méthylène pouvant être substitués respectivement par un atome d'oxygène ou de soufre, par un groupe -NH ou -N (alkyle en C₁ à C₃) ou par un groupe carbonyle, sulfonyle ou sulfonyle et
la double liaison peut être un composant d'un groupe aryle ou hétéroaryle annelé au cycle,
un groupe aryle ou
un groupe hétéroaryle,
un groupe cycloalkyle en C₃ à C₁₈, dans lequel les atomes d'hydrogène peuvent être substitués totalement ou partiellement par des atomes de fluor et dans lequel 1 à 6 groupes méthylène peuvent être substitués respectivement par un atome d'oxygène ou de soufre, par une groupe -NH ou -N(alkyle en C₁ à C₃) ou par un groupe carbonyle, sulfinyle ou sulfonyle),
un groupe alcényle en C₃ à C₂₀ dans lequel les atomes d'hydrogène peuvent être substitués totalement ou partiellement par des atomes de fluor et dans lequel 1 à 6 groupes méthylène peuvent être substituées respectivement par un atome d'oxygène ou de soufre, par un groupe NH ou -N(alkyle en C₁ à C₃) ou par un groupe carbonyle, sulfinyle ou sulfonyle),
un groupe cycloalcényle en C₅ à C₂₀ dans lequel les atomes d'hydrogène peuvent être substitués totalement ou partiellement par des atomes de fluor et dans lequel 1 à 4 groupes méthylène peuvent être substitués respectivement par un atome d'oxygène ou de soufre, par un groupe -NH ou -N(alkyle en C₁ à C₃) ou par un groupe carbonyle, sulfinyle ou sulfonyle),
et dans lequel la double liaison peut être un composant d'un groupe aryle ou hétéroaryle annelé au cycle,
un groupe alcinyle en C₅ à C₂₀ dans lequel les atomes d'hydrogène peuvent être substitués totalement ou partiellement par des atomes de fluor et dans lequel 1 à 6 groupes méthylène peuvent être substitués respectivement par un atome d'oxygène ou de souffre, par un groupe -NH ou -N(alkyle en C₁ à C₃) ou par un groupe carbonyle, sulfinyle ou sulfonyle),
un groupe aryle,
un groupe hétéroaryle,
un groupe aryl-alcényle en C₂ à C₆, ou, dans la mesure où X ne représente pas un atome d'azote qui est substitué par un groupe hydroxy, aryloxy, arylméthyloxy, hétéroaryloxy, hétéroarylméthyloxy ou alkyloxy en C₁ à C₁₀, également un groupe amino qui peut être substitué par un ou deux groupes alkyle en C₁ à C₃,
ou de même, dans la mesure où X n'est pas un atome d'azote qui est substitué par un groupe hydroxy, aryloxy, arylméthyloxy, hétéroaryloxy, hétéroarylméthyloxy ou alkyloxy en C₁ à C₁₀, également un groupe cycloalcylène-imino de 3 à 7 chaînons, un à deux groupes méthylène du groupe cycloalcylène-imino pouvant être substitué(s) respectivement par un atome d'oxygène ou un groupe carbonyle, sulfinyle ou sulfonyle, ou
R³ et X désignent conjointement un atome de fluor ou de chlore,
où les groupes aryle mentionnés dans la définition des radicaux précédemment cités désignent des groupes phényle ou naphtyle qui peuvent être substitués une à cinq fois indépendamment les uns des autres par des atomes de fluor ou de chlore et mono- di- ou trisubstitués par R_{d}, les substituants pouvant être identiques ou différents et R_{d} représente un atome de brome ou d'iode, un groupe trifluorométhyle, cyano, nitro, amino, aminocarbonyle, aminosulfonyle, méthylsulfonyle, acétylamino, méthylsulfonylamino, alkyle en C₁ à C₄, alkyle en C₁ à C₃-carbonyle, cyclopropyle, éthényle, éthinyle, hydroxy, alkyloxy en C₁ à C₄, alcoxy en C₁ à C₄-carbonyle, méthylsulfinyle, phénylsulfinyle, méthylsulfonyle, phénylsulfonyle, difluorométhoxy ou trifluorométhoxy,
les groupes hétéroaryle mentionnés dans la définition des radicaux mentionnés précédemment désignent un groupe pyrrolyle, furanyle, thiényle, pyridyle, indolyle, benzofuranyle, benzothiophényle, quinolinyle ou isoquinolinyle,
ou un groupe pyrrolyle, furanyle, thiényle ou pyridyle dans lequel un ou deux groupes méthine sont remplacés par des atomes d'azote,
ou un groupe indolyle, benzofuranyle, benzothiophényle, quinolinyle ou isoquinolinyle dans lequel un à trois groupes méthine sont remplacés par des atomes d'azote,
et les groupes hétéroaryle mentionnés précédemment peuvent être substitués une à cinq fois par des atomes de fluor ou de chlore et les R_{d} peuvent être mono-, di-ou trisubstitués,les substituants pouvant être identiques ou différents et R_{d} étant tel que défini précédemment,
et désignent, dans la définition des groupes cycloalkyle mentionnés précédemment, des systèmes cycliques mono- et également polycycliques qui sont pontés, reliés par un groupe spiro ou annelés,
ou un groupe pyrrolyle, furanyle, thiényle ou pyridyle dans lequel un ou deux groupes méthine sont remplacés par des atomes d'azote,
ou un groupe indolyle, benzofuranyle, benzothiophényle, quinolinyle ou isoquinolinyle dans lequel un à trois groupes méthine sont substitués par des atomes d'azote,
et les groupes hetéroaryle mentionnés précédemment peuvent, être substitués une à cinq fois par des atomes de fluor et de chlore et les R_{d} peuvent être mono-, di-ou trisubstitués, les substituants pouvant être identiques ou différents et R_{d} est tel que défini précédemment,
et désignent, dans la définition des groupes cycloalkyle mentionnés précédemment, des systèmes cycliques mono- et également polycyclique qui sont pontés, reliés par un groupe spiro ou annelés,
et désignent, dans la définition des groupes cycloalcényle mentionnés précédemment, des systèmes cycliques mono- et également polycycliques qui sont pontés ou annelés et qui portent au moins une double liaison C=C,
sauf indication contraire, les groupes alkyle, alcényle et alcinyle pouvant être à chaîne linéaire ou ramifiée,
la liaison dans laquelle R¹ est un atome de brome, R² est un groupe 2-butinyle, X est un atome d'oxygène et R³ est un groupe méthyle étant exclue,
leurs tautomères, leurs énantiomères, leurs diastéréoméres, leurs mélanges et leurs sels.

2. Composés de formule générale I selon la revendication 1, dans laquelle
R² et R³ sont tels que définis dans la revendication 1,
R¹ représente un atome de chlore ou de brome,
X représente un atome d'oxygène ou un groupe -NH ou -N (alkyle en C₁ à C₃),
la liaison dans laquelle R¹ est un atome de brome, R² est un groupe 2-butinyle, X est un atome d'oxygène et R³ est un groupe méthyle étant exclue,
leurs tautomères, leurs énantioméres, leurs diastéréomères, leurs mélanges et leurs sels.

3. Composés de formule générale I selon la revendication 2, dans laquelle
R¹ est un atome de chlore ou de brome,
R² est un groupe alkyle en C₁ à C₃ substitués par un groupe Rₐ, Rₐ étant tel que défini dans la revendication 1,
un groupe acétyle en C₃ à C₈,
un groupe acétyle en C₃ à C₆ substitué par un atome de fluor, de chlore ou de brome ou par un groupe trifluorométhyle ou
un groupe alcinyle en C₃ à C₈,
X est un atome d'oxygène et
R³ est un atome d'hydrogène,
un groupe alkyle en C₁ à C₂₀ dans lequel les atomes d'hydrogène peuvent être substitués totalement ou partiellement par des atomes de fluor et dans lequel 1 à 6 groupes méthylène peuvent être substitués respectivement par un atome d'oxygène ou de soufre, par un groupe -NH ou -N (alkyle en C₁ à C₃) ou par un groupe carbonyle, sulfinyle ou sulfonyle,
un groupe alkyle en C₁ à C₁₂ substitué par un groupe R_{c} dans lequel les atomes d'hydrogène peuvent être substitués totalement ou partiellement par des atomes de fluor et dans lequel 1 à 4 groupes méthylène peuvent être substitués respectivement par un atome d'oxygène ou de soufre, par un groupe -NH ou N (alkyle en C₁ à C₃) ou par un groupe carbonyle, sulfinyle ou sulfonyle, et R_{c} étant tel que défini précédemment,
un groupe cycloalkyle en C₃ à C₈ dans lequel les atomes d'hydrogène peuvent être substitués totalement ou partiellement par des atomes de fluor et dans lequel 1 à 4 groupes méthylène peuvent être substitués respectivement par un atome d'oxygène ou de soufre, par un groupe -NH ou -N(alkyle en C₁ à C₃) ou par un groupe carbonyle, sulfinyle ou sulfonyle,
un groupe alcényle en C₃ à C₂₀ dans lequel les atomes d'hydrogène peuvent être substitués totalement ou partiellement par des atomes de fluor et dans lequel 1 à 6 groupes méthylène peuvent être substitués respectivement par un atome d'oxygène ou de soufre, par un groupe -NH ou -N(alkyle en C₁ à C₃) ou par un groupe carbonyle, sulfinyle ou sulfonyle,
un groupe alcinyle en C₃ à C₂₀ dans lequel les atomes d'hydrogène peuvent être substitués totalement ou partiellement par des atomes de fluor et dans lequel 1 à 6 groupes méthylène peuvent être substitués respectivement par un atome d'oxygène ou de soufre, par un groupe NH ou -N(alkyle en C₁ à C₃) ou par un groupe carbonyle, sulfinyle ou sulfonyle, ou
un groupe aryl-alcényle en C₂ à C₆,
la liaison dans laquelle R¹ est un atome de brome, R² est un groupe 2-butinyle, X est un atome d'oxygène et R³ est un groupe méthyle étant exclue,
leurs tautomères, leurs énantiomères, leurs diastéréomères, leurs mélanges et leurs sels.

4. Composés de formule générale I selon la revendication 3, dans laquelle
R¹ est un atome de chlore ou de brome,
R² est un groupe phénylméthyle qui peul être substitué au niveau du cycle phényle, par un atome de fluor, de chlore, de brome ou d'iode ou par un groupe cyano ou méthoxy,
un groupe alcényle en C₃ à C₈,
un groupe cycloalcénylméthyle en C₃ à C₈,
un groupe alcényle en C₃ à C₆ qui peut être substitué par un atome de fluor, de chlore ou de brome ou par un groupe trifluorométhyle,
un groupe alcinyle en C₃ à C₈,
X représente un atome d'oxygène et
R³ représente un atome d'hydrogène,
un groupe alkyle en C₁ à C₂₀ dans lequel les atomes d'hydrogène peuvent être substitués totalement ou partiellement par des atomes de fluor et dans lequel 1 à 6 groupes méthylène peuvent être substitués respectivement par un atome d'oxygène ou de soufre, par un groupe NH ou N(alkyle en C₁ à C₃),
un groupe alkyle en C₁ à C₁₂ substitué par un groupe R_{c} dans lequel les atomes d'hydrogène peuvent être substitués totalement ou partiellement par des atomes de fluor et dans lequel 1 à 4 groupes méthylène peuvent être substitués respectivement par un atome d'oxygène ou de soufre, ou par un groupe -NH ou N (alkyle en C₁ à C₃) et dans lequel R_{c} est tel que défini précédemment,
un groupe cycloalkyle en C₃ à C₈ dans lequel les atomes d'hydrogène peuvent être substitués totalement ou partiellement par des atomes de fluor et dans lequel 1 à 4 groupes méthylène peuvent être substitués respectivement par un atome d'oxygène ou de soufre, par un groupe -NH ou -N(alkyle en C₁ à C₃),
un groupe alcényle en C₃ à C₂₀ ou
un groupe alcinyle en C₃ à C₂₀,
la liaison dans laquelle R¹ est un atome de brome, R² est un groupe 2-butinyle, X est un atome d'oxygène et R³ est un groupe méthyle étant exclue,
leurs tautomères, leurs énantiomères, leurs diastéréomères, leurs mélanges et leurs sels.

5. Composés de formule générale I selon la revendication 4, dans laquelle
R¹ représente un atome de brome,
R² représente un groupe 2-butén-1-yle ou 3-méthyl-2-butén-1-yle,
un groupe 2-butin-1-yle ou
un groupe 2-chlorophénylméthyle ou 2-bromo-phénylméthyle,
X représente un atome d'oxygène et
R³ représente un groupe alkyle en C₁ à C₁₀ ou cycloalkyle en C₃ à C₈,
la liaison dans laquelle R¹ est un atome de brome, R² est un groupe 2-butinyle, X est un atome d'oxygène et R³ est un groupe méthyle étant exclue,
leurs tautomères, leurs énantiomères, leurs diastéréomères, leurs mélanges et leurs sels.

6. Composés suivants de formule générale I selon la revendication 5 :
(a) acide 2-bromo-5-formyl-3-(3-méthyl-2-butén-1-yl)-3H-imidazol-4-carboxylique méthylester,
(b) acide 2-bromo-5-formyl-3-(2-butin-1-yl)3H-imidazol-4-carboxylique éthylester et leurs sels.

7. Procédé de production des composés I selon la revendication 1, **caractérisé en ce que**
a) un composé de formule générale 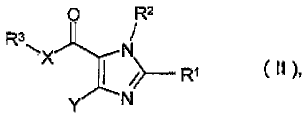 dans laquelle
R¹ à R³ et X sont tels que définis dans les revendications 1 à 6 et Y représente un groupe acide carboxylique, amide d'acide carboxylique, nitrile, ester d'acide carboxylique, thioester d'acide carboxylique, anhydride d'acide carboxylique ou chlorure d'acide carboxylique qui sont liés respectivement par l'atome de carbone du carboxyle au cycle imidazole, est réduit
b) un composé de formule générale 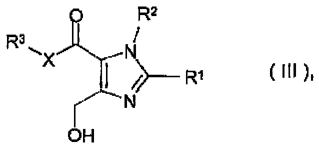 dans laquelle R¹, R², R³ et X sont tels que définis dans les revendications 1 à 6, est oxydé.

8. Procédé de production d'imidazo[4,5-d]pyridazin-4-ones de formule générale 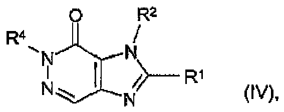 dans laquelle R¹ et R² sont tels que définis dans les revendications 1, à 6,
**caractérisé en ce qu'**un composé de formule générale I, dans laquelle R¹ à R³ et X sont tels que définis dans les revendications 1 à 6, est converti avec une hydrazine substituée par R⁴ ou un dérivé d'hydrazine protégé de façon correspondante,
dans laquelle
R⁴ represente un atome d'hydrogène,
un groupe alkyle en C₃ à C₈,
un groupe allyle en C1 a C3 substitué par un groupe Rₐ, Rₐ étant tel que défini dans la revendication 1,
un groupe alcényle en C₃ à C₈ ou alcinyle on C₃ à C₈,
un groupe arylcarbonylméthyle ou hétéroaryl-carbonylméthyle,
un groupe arylprop-2-ényle ou hétéroarylprop-2-ényle ou un groupe aryle ou hétéroaryle,
et éventuellement, les radicaux R¹ et/ou R² du composé ainsi obtenu de formule générale IV sont modifiés ou d'autres substituants sont introduits dans La structure fondamentale
et si on le souhaite, le composé de formule IV est converti en ses sels avec des acides ou des bases organiques et anorganiques.

9. Procédé de production d'imidazo[4,5-d]pyridazin-4-ones de formule générale 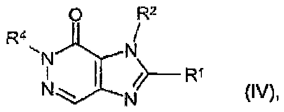 dans laquelle R¹ et R² sont tels que définis dans les revendications 1 à 6,
**caractérisé en ce que**
a) un compose de formule générale 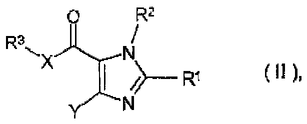 dans laquelle
R¹ a R³ et X sont tels que définis dans les revendications 1 à 6, et Y représente un groupe acide carboxylique, amide d'acide carboxylique, nitrile, ester d'acide carboxylique, thioester d'acide carboxylique, anhydride d'acide carboxylique ou chlorure d'acide carboxylique qui sont liés respectivement par l'atome de carbone du carboxyle au cycle imidazole, est réduit
ou
b) un composé de formule générale 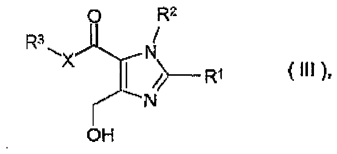
dans laquelle R¹, R², R³ et X sont tels que définis dans les revendication 1 à 6, est oxydé;
et c) un composé obtenu selon l'étape a) ou b) de formule générale 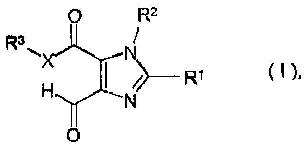 dans laquelle R' à R³ et X sont tels que définis dans les revendication 1 à 6, est converti avec une hydrazine substituée par R⁴ ou un dérivé d'hydrazine protégée correspondant,
R⁴ représentant
un atome d'hydrogène,
un groupe alkyle on C₃ à C₈,
un groupe alkyle en C₁ à C₃ substitué par un groupe Rₐ, Rₐ étant tel que défini à la revendication 1,
un groupe alcényle en C₃ à C₈ ou alcinyle en C₃ à C₈,
un groupe arylcarbonylméthyle ou hétéroaryl-carbonylméthyle,
un groupe arylprop-2-ényle ou hétéroarylprop-2-ényle ou
un groupe aryle ou hétéroaryle,
et éventuellement, les radicaux R¹ et/ou R² du composé ainsi obtenu de formule générale IV sont modifiés ou d'autres substituants sont introduits dans la structure fondamentale
et si on le souhaite, le composé de formule IV est converti en ses sels avec des acides ou des bases organiques et anorganiques.
